# EUROPEAN PATENT APPLICATION

(11) **EP 3 372 244 A2**
(43) Date of publication of application: **12.09.2018**
(21) Application number: 18154502.1
(22) Date of filing: 27.10.2011
(51) Int. Cl.: A61K 39/395

(54) **CHIMERIC CD27 RECEPTORS FOR REDIRECTING T CELLS TO CD70-POSITIVE MALIGNANCIES**

(30) Priority: 27.10.2010 US 407189 P
(62) Divisional of application: 11837112.9
(71) Applicant: Baylor College of Medicine, Houston, TX 77030-3411 (US)
(72) Inventor: GOTTSCHALK, Stephen M. G, Houston, TX 77030 (US); SCHAFFER, Donald R., Boston, MA 02111 (US); SPENCER, David M., Houston, TX 77025 (US)
(74) Representative: J A Kemp

(57) **Abstract**

The present invention concerns methods and compositions related to T cells redirected against CD70 for the immunotherapy of CD70-positive malignancies. In aspects of the invention, T cells that are CD70-specific are employed. In particular aspects, there are T cells expressing a novel molecule that comprises the full-length CD70 receptor (CD27) fused to the zeta signaling domain of the T-cell receptor complex. Such T cells recognized CD70-positive tumor cells and have cytolytic activity against CD70-positive cancer cells.

## Description

### CROSS-REFERENCE TO RELATED APPLICATIONS

This application claims priority to U.S. Provisional Patent Application Serial No. 61/407,189, filed on October 27, 2010, which is incorporated by reference herein in its entirety.

### STATEMENT REGARDING FEDERALLY SPONSORED RESEARCH OR DEVELOPMENT

This invention was made with government support under PO1 CA094237 awarded by NIH/NCI and under T32 DK64717 awarded by NIH/NIDDK and under 5T32HL092332-07 awarded by NIH. The government has certain rights in the invention.

### TECHNICAL FIELD

Embodiments of the present invention concern the fields of cell biology, molecular biology, immunology, and medicine.

### BACKGROUND OF THE INVENTION

Immunotherapy with antigen-specific T cells has shown promise in the treatment of hematological malignancies in preclinical models as well as in Phase I/II clinical studies. (Leen *et al*., 2007; Bollard *et al*., 2007; June, 2007; Rosenberg *et al*., 2008; Di Stasi *et al*., 2009; Vera *et al*., 2006) One attractive strategy to generate tumor-specific T cells is by genetic modification with chimeric antigen receptors (CARs), which consist of an extracellular antigen recognition domain, a transmembrane domain, and an intracellular signaling domain derived from the T-cell receptor CD3-δ chain often linked to costimulatory molecule endodomains. (Rossig and Brenner, 2004; Sadelain *et al*., 2003) CARs targeting CD19 and CD20 antigens for the treatment of hematological malignancies have been explored extensively, but this approach is limited to B-cell derived malignancies and may produce prolonged impairment of humoral immunity because of the potentially long life span of T cells. (Till *et al*., 2008; Cooper *et al*., 2005) It is therefore desirable to prepare CARs directed against alternative antigens that could broaden the spectrum of potentially treatable tumors and/or reduce damage to normal cells.

CD70 is the membrane bound ligand of the CD27 receptor, which belongs to the tumor necrosis factor receptor superfamily. (Hintzen *et al*., 1994; Bowman *et al*., 1994) CD70 is expressed by diffuse large B-cell and follicular lymphoma and also by the malignant cells of Hodgkin's lymphoma, Waldenstrom's macroglobulinemia and multiple myeloma, and by HTLV-1- and EBV-associated malignancies. (Agathanggelou et al,. 1995; Hunter *et al*., 2004; Lens *et al*., 1999; Baba *et al*., 2008) In addition, CD70 is expressed by non-hematological malignancies such as renal cell carcinoma and glioblastoma. (Junker *et al*., 2005; Chahlavi *et al*., 2005) Physiologically, CD70 expression is transient and restricted to a subset of highly activated T, B, and dendritic cells. While CD70/CD27 costimulation plays a role in T-cell activation, CD70/CD27 signaling is not essential for the development and maintenance of a functional immune system since CD27 knockout mice have no overt immunodeficiency and recover from influenza virus infection within the same time frame as wild type mice. (Hendriks *et al*., 2000; Nolte *et al*., 2009)

### BRIEF SUMMARY OF THE INVENTION

The present invention is directed to methods and/or compositions that concern immunotherapy for the treatment and/or prevention of cancer. In specific aspects, embodiments of the invention concern T cells redirected against CD70 for the immunotherapy of CD70-positive cells, including malignancies for example. The invention may be employed for any mammal, male or female, including humans, dogs, cats, horses, and so forth.

Expression of CD70, a member of the tumor necrosis factor superfamily, is restricted to activated T- and B-lymphocytes and mature dendritic cells. Binding of CD70 to its receptor, CD27, is important in priming, effector functions, differentiation and memory formation of T-cells as well as plasma and memory B-cell generation. In particular, CD70 is expressed on a broad spectrum of a) hematological malignancies, such as multiple myeloma, non-Hodgkin's lymphomas and Hodgkin's disease, for example; b) solid tumors, such as renal cell carcinoma, pancreatic, ovarian, lung and nasopharyngeal carcinoma, and c) brain tumors, such as glioblastoma mutliforme, for example. Preclinical studies in animal models using monoclonal antibodies have validated CD70 as an immunotherapeutic target. The inventors have now redirected T cells with a genetic approach to CD70-positive malignancies. For this purpose the inventors have constructed a novel molecule (CD27zeta) that consists of the full-length CD70 receptor (CD27) fused to the zeta signaling domain of the T-cell receptor complex. T cells expressing CD27zeta were generated by retroviral transduction, and CD27zeta expressing T cells recognized CD70-positive tumor cells as judged by their abilitv to proliferate and produce IFN-γ as well as IL-2 in contrast to non-transduced T cells after coculture with CD70-positive tymor cells. In addition, CD27zeta expressing T cells had cytolytic activity and killed CD70-positive tumor cells, whereas CD70-negative tumor cells were not killed.

In one embodiment of the invention, there are methods for reducing or preventing tumors comprising introducing a nucleic acid construct encoding an chimeric receptor if the invention into an isolated T cell of an individual having or suspected of having a tumor and delivering (such as by injection) the T cell into the individual so that the chimeric receptor is expressed on the surface of the T cell to activate anti-tumor immunity in the individual, thereby reducing or preventing the tumor.

In one embodiment of the invention, there are chimeric antigen receptors that recognizes the CD70 antigen and that comprises an intracellular signaling domain. In specific embodiments, the receptor is present on a cell, such as a T cell. In specific embodiments, the receptor is further defined as a CD70 receptor, such as CD27, for example. In certain embodiments, the intracellular signaling domain is the T-cell receptor CD3-ζ chain.

In some embodiments of the invention, there are methods of targeting a cell having a CD70 antigen, comprising the steps of providing to the cell another cell comprising a chimeric receptor of the invention. In specific embodiments, the cells being targeted may be any kind of cell that comprises a CD70 antigen, including cancer cells, and in specific embodiments they are hematological malignant cells for example. In certain aspects they are lymphoma cells, renal cell carcinoma cells, or gliobastoma cells, for example. In some aspects the cancer cells are HTLV-1-associated malignant cells or EBV-associated malignant cells, for example. In specific embodiments, the cancer cells are CD70-positive. In specific embodiments, the cancer being treated is renal cell cancer, thymic carcinoma, nasopharyngeal carcinoma, brain tumor, Hodgkin and non-Hodgkin lymphomas, Waldenstrom's macroglobulinemia, chronic lymphocytic leukemia, T-cell leukemia, multiple myeloma, EBV- and HTLV-I associated malignancies, kidney, pancreatic, larynx, pharynx, melanoma, ovarian, lung (including lung adenocarcinoma), colon, breast, or brain.

In specific embodiments of the invention, the T cell comprising the chimeric receptor targets any cell that comprises a CD70 antigen, whether or not that targeted cell is cancerous. For example, in some embodiments CD70 is expressed on cells that are related to autoimmune disorders, as in certain aspects associated with the invention there is dysregulation of CD70-CD27 co-stimulation that contributes to autoimmunity. In specific embodiments, the CD70 cells are present in an individual with an autoimmune disorder such as rheumatoid arthritis (RA), arthritis (including psoriatic arthritis), inflammation, autoimmune encephalitis, inflammatory bowel disease, colitis, and lupus.

In one embodiment of the invention , there are methods of treating a CD70-positive malignant cells in an individual, comprising the step of targeting the CD70-positive malignant cells with a tumor-specific T cell that comprises a chimeric antigen receptor of the invention. In specific embodiments, the individual has received or is receiving or will receive an additional anti-cancer therapy, such as surgery, radiation, chemotherapy, immunotherapy, or hormone therapy, for example.

The foregoing has outlined rather broadly the features and technical advantages of the present invention in order that the detailed description of the invention that follows may be better understood. Additional features and advantages of the invention will be described hereinafter which form the subject of the claims of the invention. It should be appreciated by those skilled in the art that the conception and specific embodiment disclosed may be readily utilized as a basis for modifying or designing other structures for carrying out the same purposes of the present invention. It should also be realized by those skilled in the art that such equivalent constructions do not depart from the spirit and scope of the invention as set forth in the appended claims. The novel features which are believed to be characteristic of the invention, both as to its organization and method of operation, together with further objects and advantages will be better understood from the following description when considered in connection with the accompanying figures. It is to be expressly understood, however, that each of the figures is provided for the purpose of illustration and description only and is not intended as a definition of the limits of the present invention.

### BRIEF DESCRIPTION OF THE DRAWINGS

For a more complete understanding of the present invention, reference is now made to the following descriptions taken in conjunction with the accompanying drawing, in which:

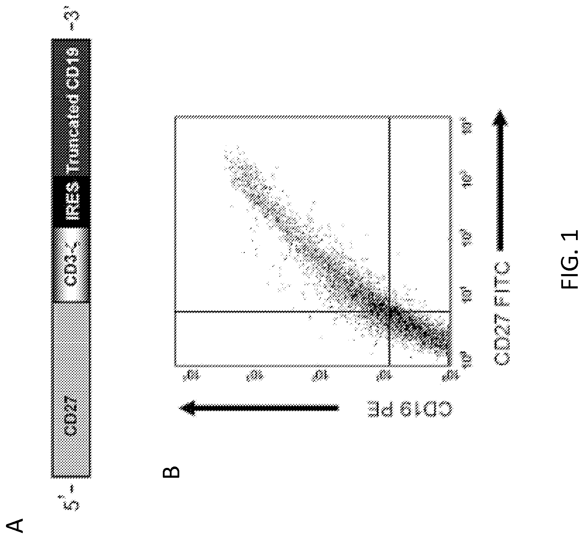

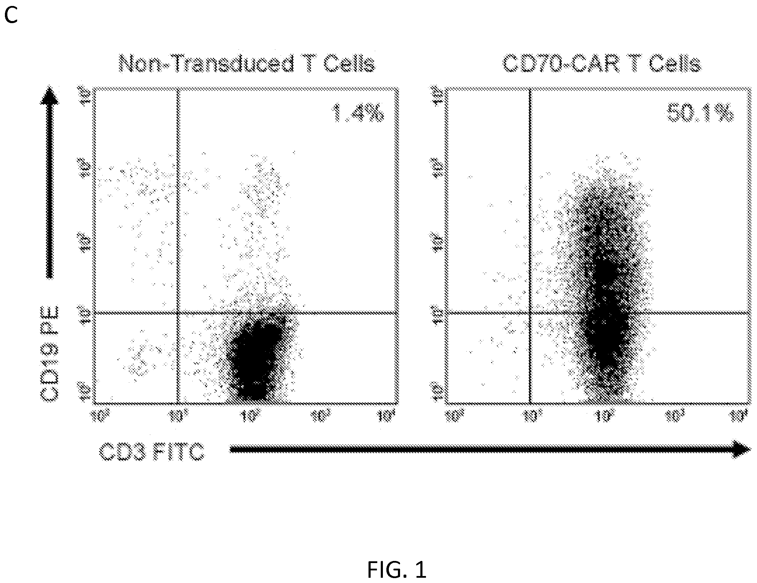

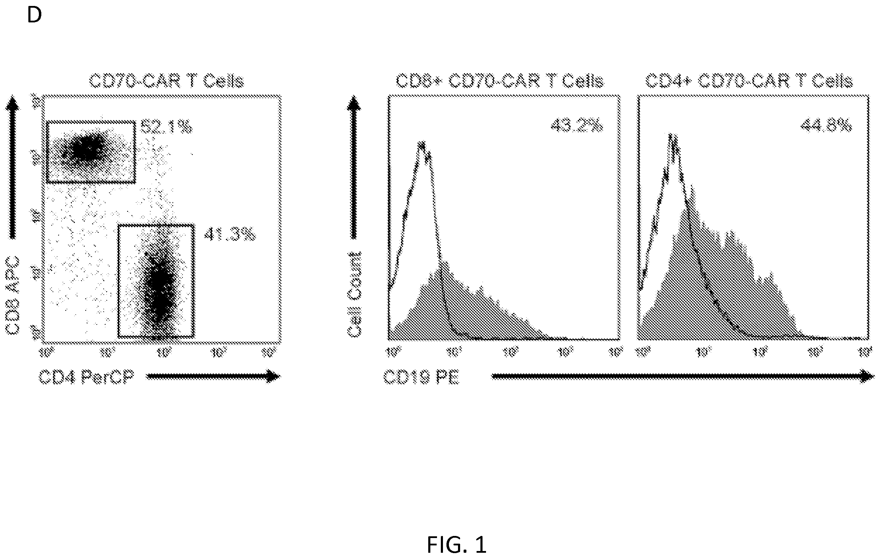

Figure 1: CD70-CAR generation, cell-surface expression, and transduction of human T cells. (A) CD70-CAR was generated by fusing full length CD27 to the signaling domain of CD3-ζ chain, an IRES sequence and tCD19 was included for detection of genetically modified T cells. (B) 293T cells transfected with CD70-CAR constructs express both CD27 and the marker gene tCD19.(C) CD70-CAR expression on transduced human T cells was 45% (+/-6) as determined by staining tCD19. (D) Both CD4 and CD8 T cells were genetically modified.

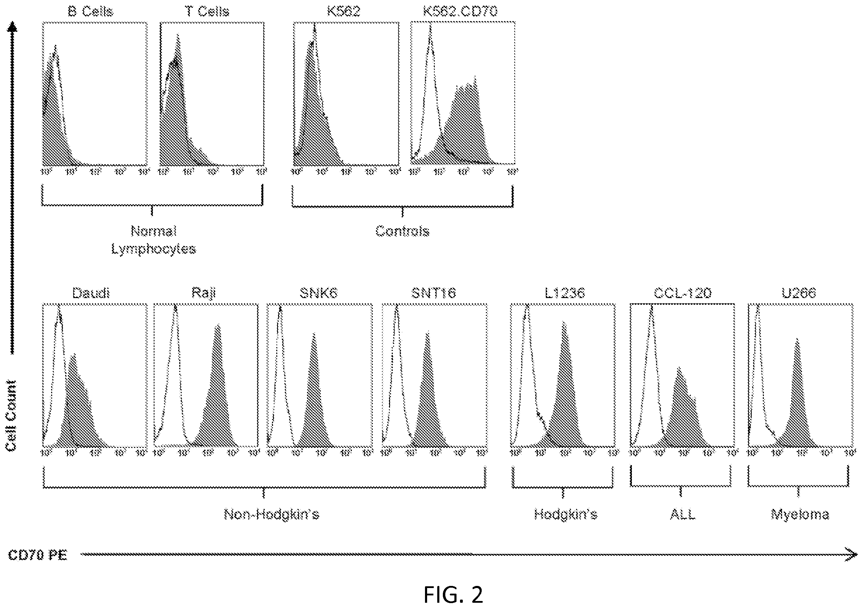

Figure 2: CD70 is overexpressed on several tumor cell lines but not normal lymphocytes. Less than 5% of B and T lymphocytes from the peripheral blood of healthy donors express CD70. K562 and K562.70 served as negative and positive controls. CD70 overexpression was observed on Non-Hodgkin's (Daudi, SNK6, SNT16), Hodgkin's (L1236), ALL (CCL-120), and Multiple Myeloma (U266) cells.

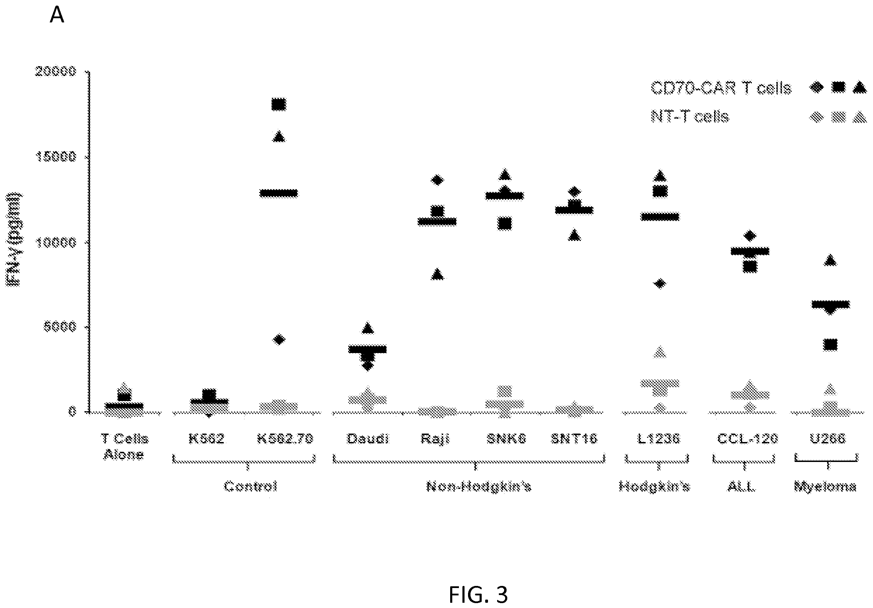

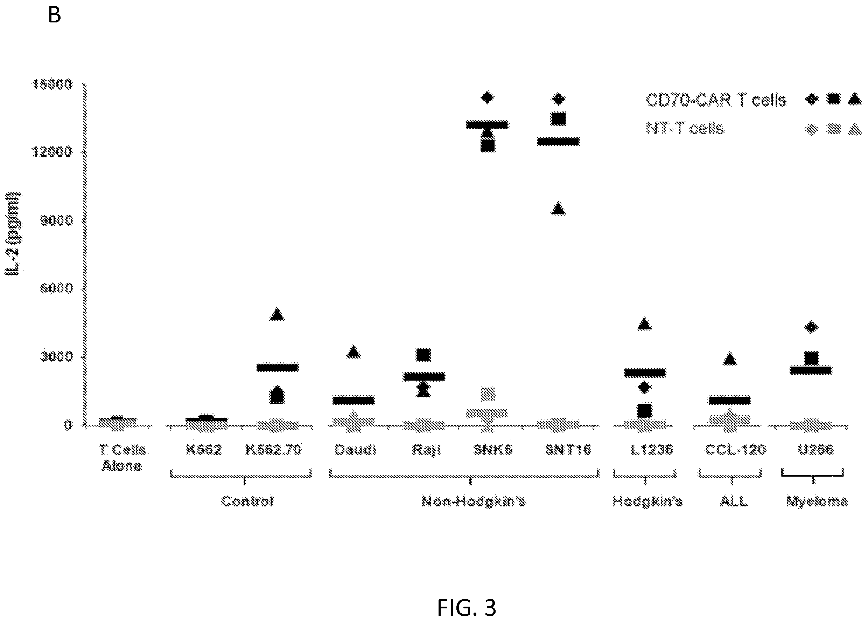

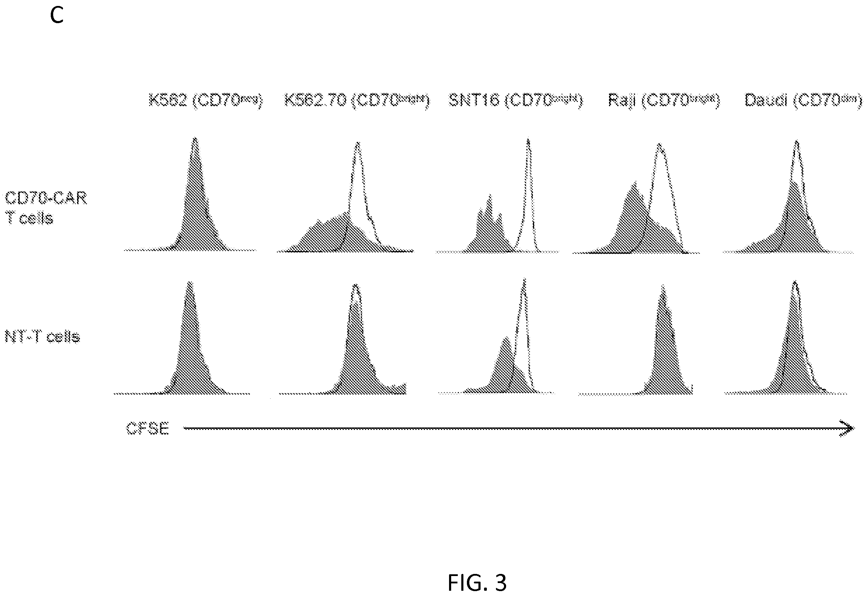

Figure 3: CD70-specific T cells release IFN-γ, IL-2 and proliferate in response to CD70-positive target cells. (A) T cells from 3 donors were transduced with CD70-CAR (black) or non-transduced (gray) and co-cultured with K562.70 and K562 as well as various CD70-expressing tumor cell lines for 48 h before performing IFN-γ ELISA. Black and gray rectangles represent mean IFN-γ release of CD70-CAR transduced or nontransduced T cells, respectively. CD70-CAR T cells were specific for CD70 as significantly (p<0.03) more IFN-γ was released in the presence of K562.70 compared to K562 cells. CD70-CAR T cells also released significantly (p<0.0001) more IFN-γ than non-transduced T cells when co-cultured with CD70-expressing tumor cell lines. (B) Same co-culture experiments but assayed for the presence of IL-2. CD70-CAR T cells release significantly (p<0.0001) more IL-2 than non-transduced T cells in the presence of CD70-expressing tumors. (C) T cells were labeled with CFSE and co-cultured for 5 days with K562, K562.70, SNT16, or Daudi in the absence of exogenous IL-2 and CFSE dilution was analyzed by flow cytometry. CD70-CAR T cells proliferated when cocultured with CD70 overexpressing targets K562.70 and SNT16 but not the CD70-dim Daudi cells or CD70-negative K562 cells.

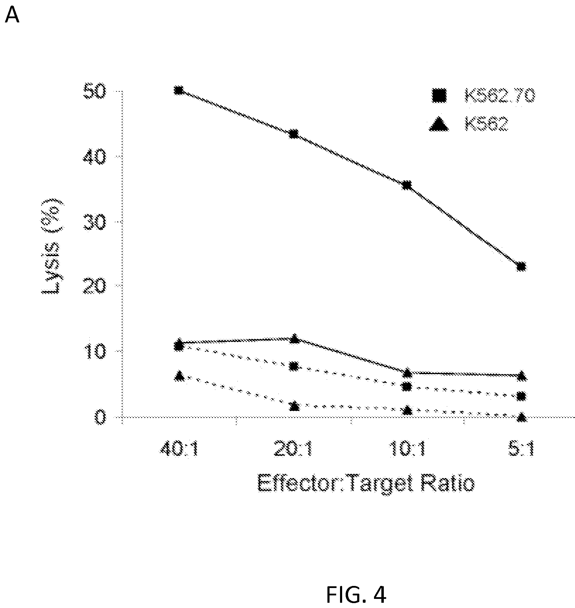

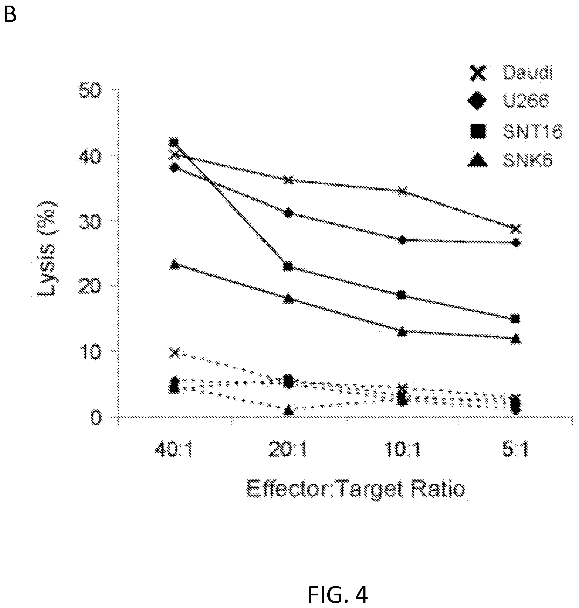

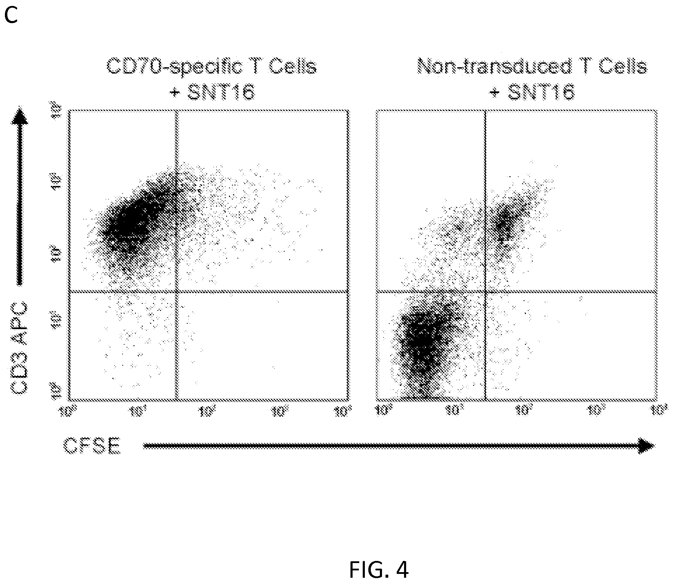

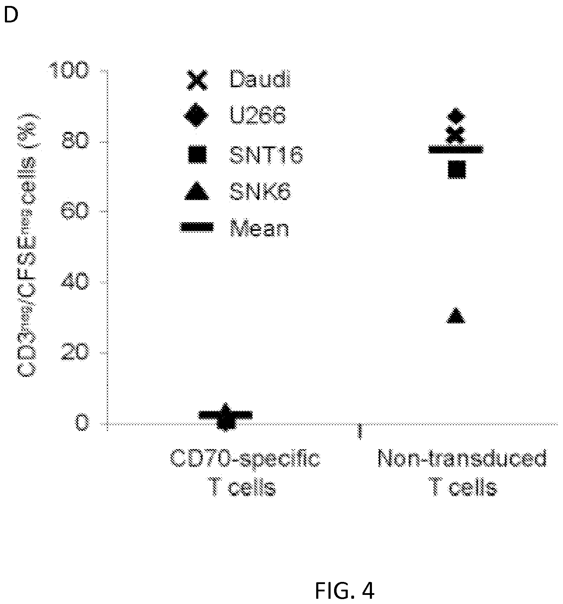

Figure 4: CD70-specific T cells kill CD70-positive tumor cell lines. (A) CD70-CAR T cells (solid lines) killed K562.70 cells but not parental K562 cells. Non-transduced control T cells (dashed lines) did not kill either target. (B) CD70-CAR T cells (solid lines) killed CD70-positive Daudi, U266, SNK6, and SNT16 tumor cell lines; control T cells (dashed lines) did not. (C) CD70-specific T cells or nontransduced T cells were labeled with CFSE and co-cultured with SNT16 cells at a ratio of 2:1. CD70-specific T cells proliferated and killed SNT16 cells as shown by CFSE dilution of CD3⁺ cells and the lack of CD3/CFSE-negative cells in the culture compared with non-transduced T cells. (D) In all coculture experiments only CD70-specific T cells eliminated the CD3/CFSE-negative CD70⁺ tumor cells Daudi, U266, SNK6, and SNT16.

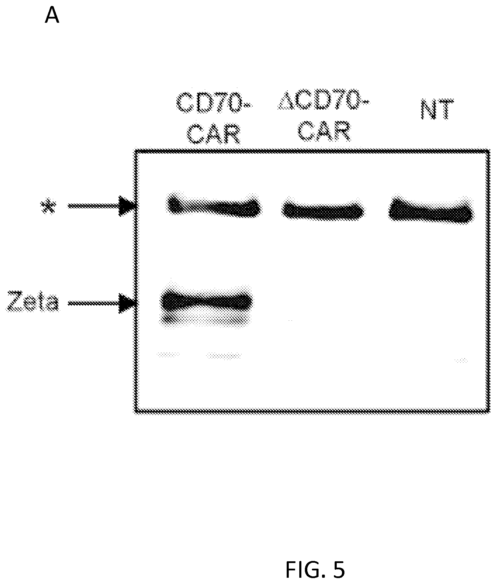

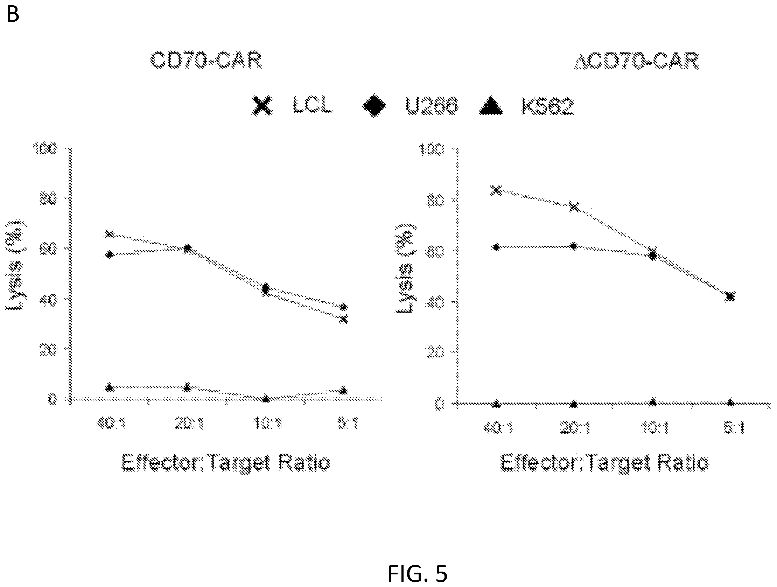

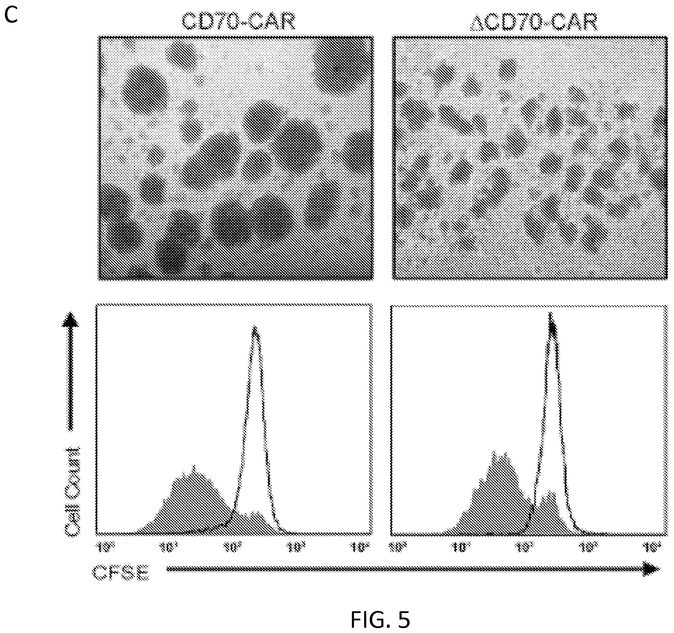

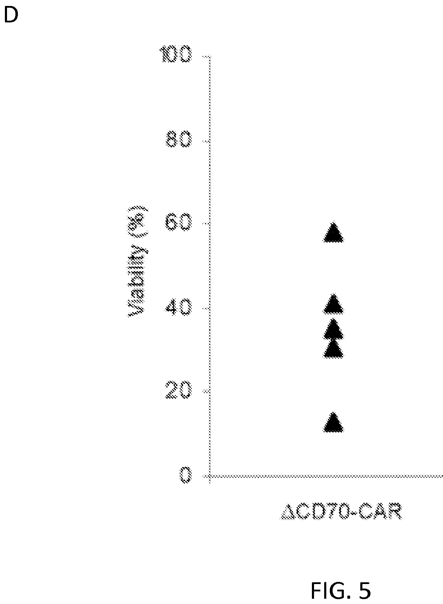

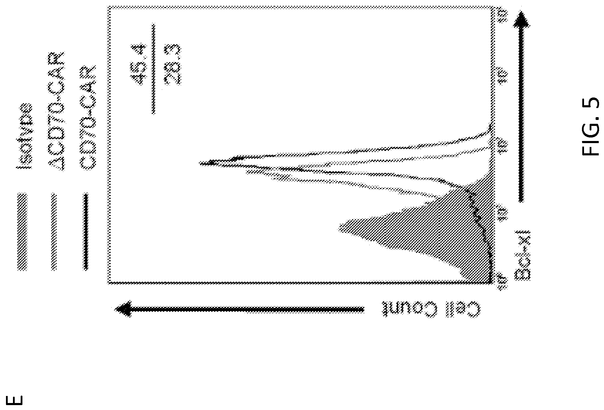

Figure 5: CD27 costimulation enhances T-cell viability. (A) In Co-IP experiments only full length CD27-ζ associated with TRAF2. (B) T cells expressing CD70-CAR or ΔCD70-CAR showed equivalent killing of CD70+ LCL and U266 cells but did not kill CD70- K562 cells in 51Cr release assays. (C) Microscopic evaluation (10X) of T cells expressing CD70-CAR or ΔCD70-CAR activated with autologous fibroblasts genetically modified to express CD70 revealed larger 'T-cell clumps' of T cells expressing CD70- CAR, however CFSE dilution analysis showed no significant differences in proliferation between groups. (D) The viability of ΔCD70-CAR T cells was 35% (+/- 16%) that of T cells expressing CD70-CAR (n=5). (E) Intracellular staining for Bcl-xl was performed on T cells 3 days after stimulation with CD70 transgenic autologous fibroblasts. Bcl-xl expression was consistently increased in CD70-CAR T cells compared with ΔCD70-CAR T cells (n=3). One representative FACS analysis is shown).

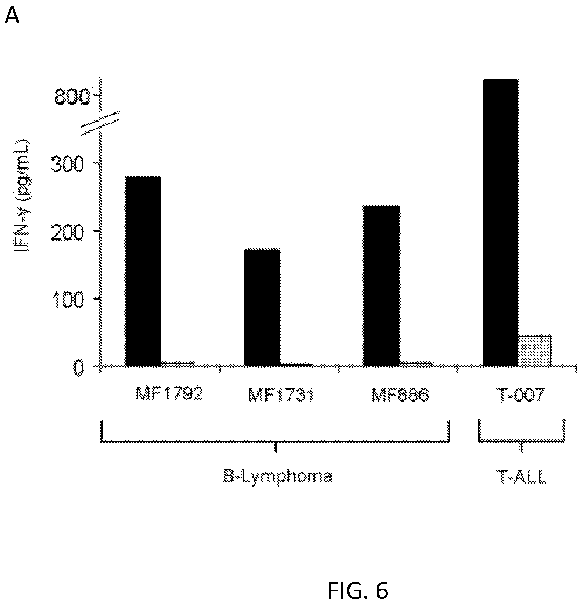

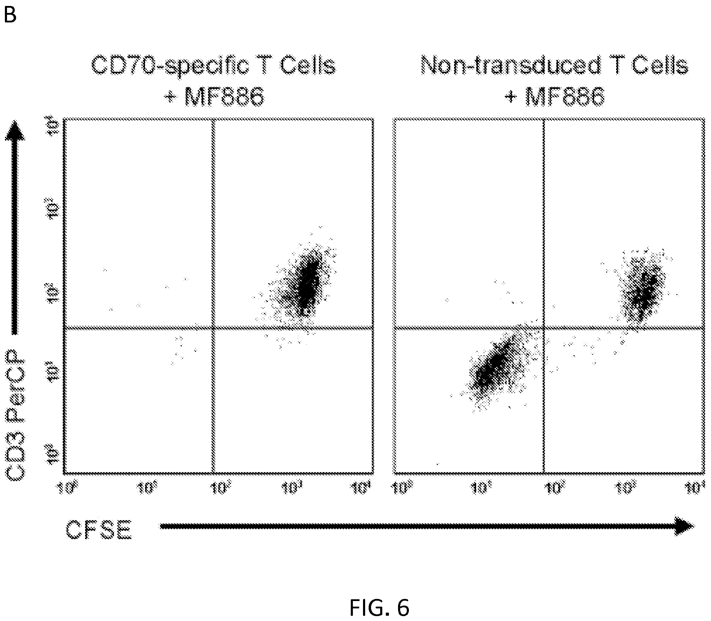

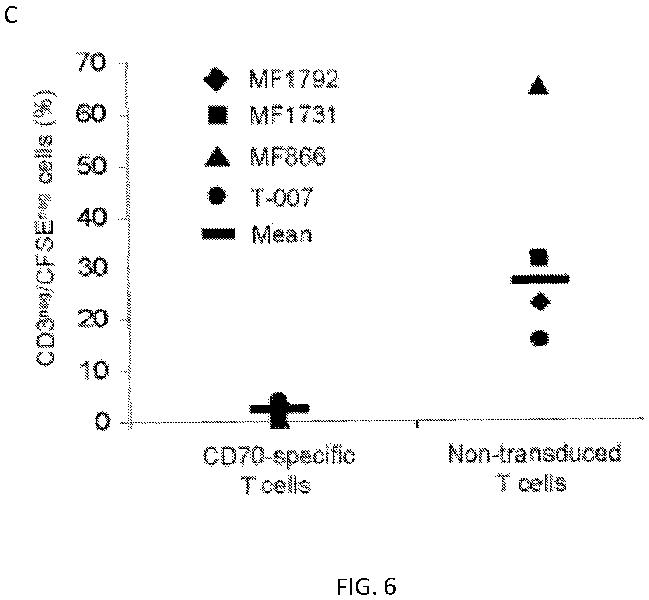

Figure 6: CD70-specific T cells recognize and kill primary CD70-positive lymhomas. (A) CD70 overexpressing tumor cells from 3 patients with B-cell lymphoma and 1 patient with T-cell acute lymphoblastic leukemia were cocultured with CD70- specific or non-transduced T cells from healthy donors for 48h before performing IFN-γ ELISA. In all cases CD70-specific T cells released IFN-γ in the presence of patient tumor cells whereas non-transduced cells released little to no IFN-γ. (B, C) Coculture assays were performed with primary tumor cells and CFSE labeled T cells to distinguish effector and target cells by FACS analysis. Only CD70-specific T cells (CD3/CFSE positive cells) were able to eradicate patient tumor cells (p=0.036).

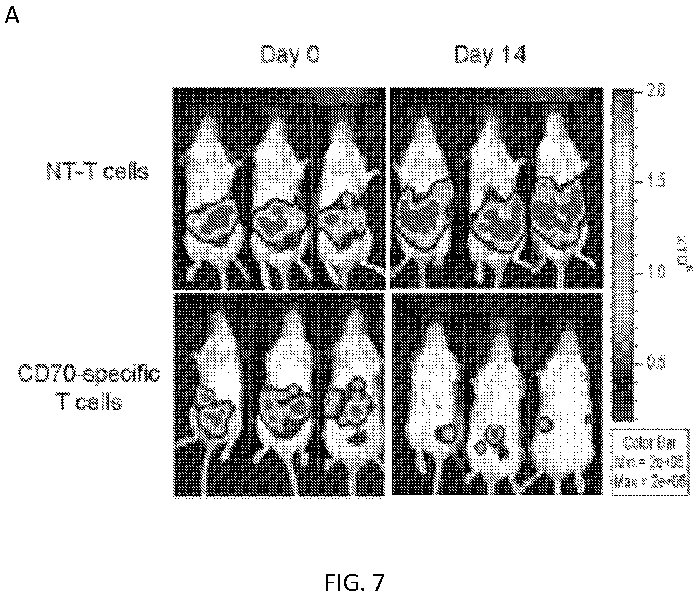

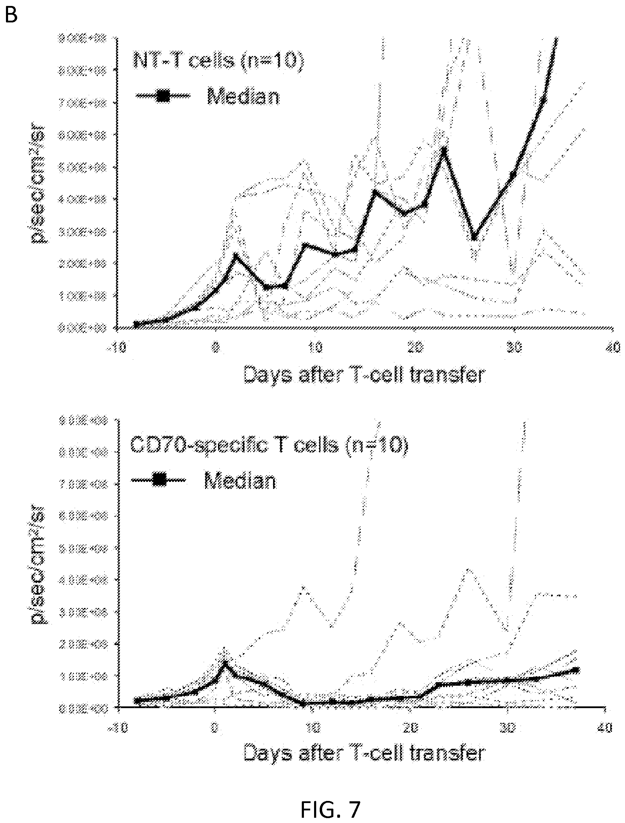

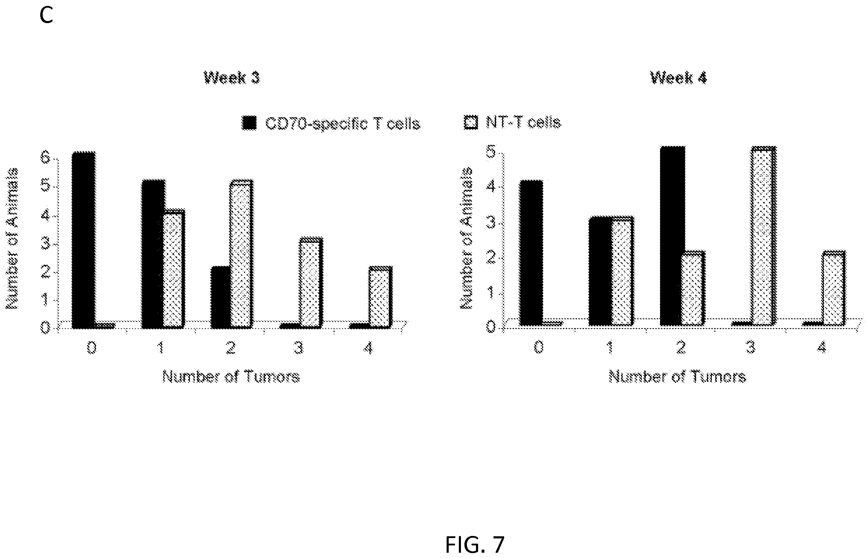

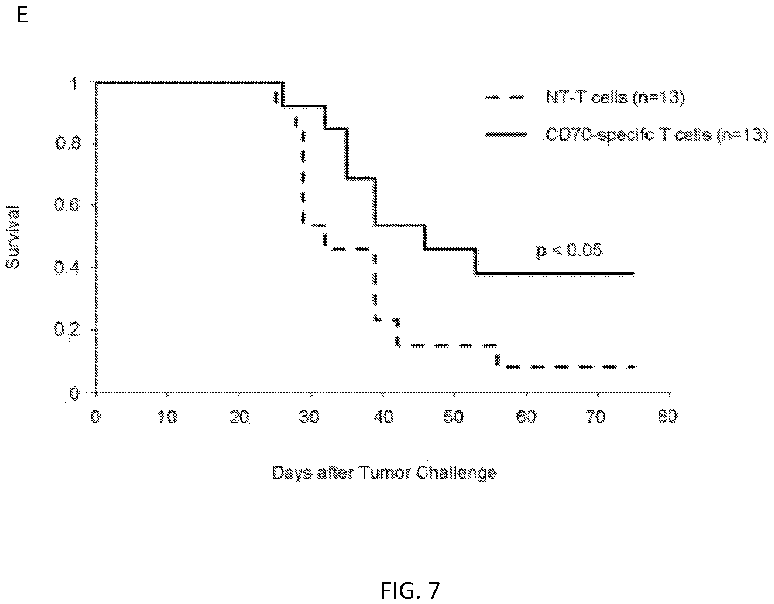

Figure 7: CD70-specific T cells exhibit *in vivo* anti-tumor activity in a murine xenograft model of lymphoma. (A-B) Daudi cells (5 x 10⁵) expressing eGFP-FFLuc gene were injected intraperitoneally into SCID mice. Tumor growth was measured as increasing light signal (photon/sec/cm²/sr). On day 10, 11 and 17 mice were injected with 1x10⁷ CD70-specific or non-transduced T cells. Tumors treated with CD70-specific T cells regressed, whereas tumors treated with non-transduced T cells did not (*P*=0.002) at 7 day post treatment). Panel A shows images of representative animals. Panel B shows quantitative bioluminescence imaging. In panels C and D, Raji cells (2 x 10⁵) were injected intravenously into SCID mice. On days 4, 5, and 11, mice were injected with 1 x 10⁷ CD70-specific or nontransduced T cells. (C) Systemic tumors were enumerated using bioluminescence imaging. At weeks 3 and 4 after tumor cell injection, there was a significantly higher tumor burden in mice receiving nontransduced T cells than CD70-specific T cells (week 3, P = .012; week 4 [n = 12], *P* .010). (D) Mice treated with CD70-specific T cells displayed a significant survival advantage over those receiving nontransduced T cells (P < .05).

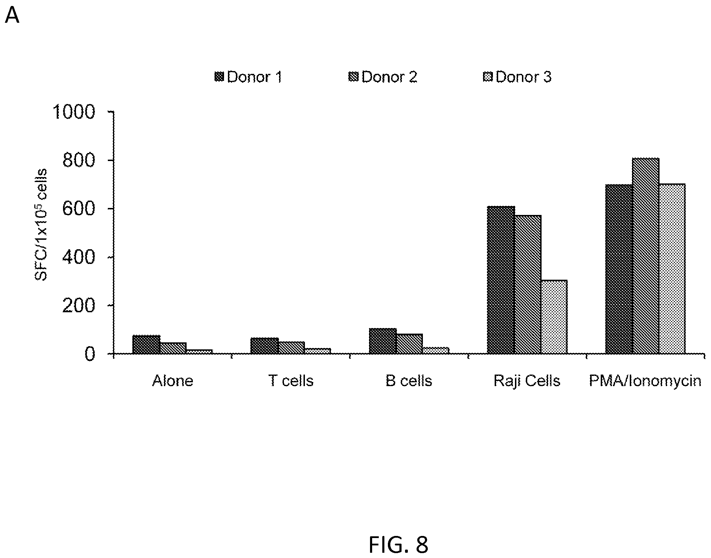

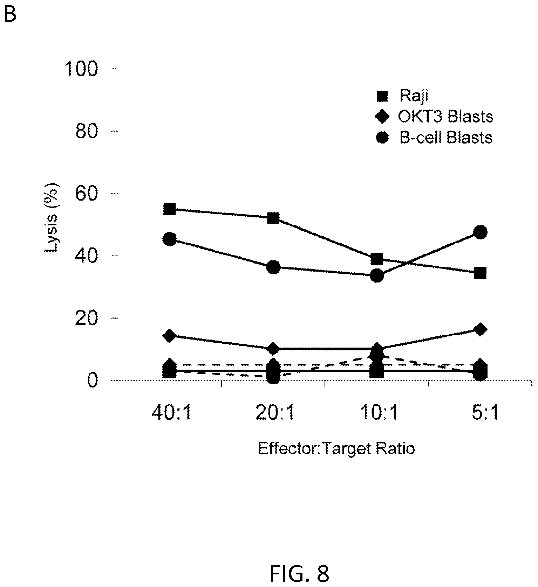

Figure 8. CD70-specific T cells show minimal reactivity against autologous B and T cells. (A) CD70-specific T cells (1 x 105) from 3 healthy donors were plated alone, in the presence of 5 x 104 autologous T cells, B cells, or Raji cells, or stimulated with PMA/ionomycin. Strong reactivity is seen against Raji cells and after PMA/ionomycin treatment, but not against autologous T or B cells, as measured by IFN-γ ELISPOT. (B) CD70-specific T cells kill Raji cells and B-cell blasts, but not OKT3 blasts in a 4 h 51chromium release assay. Non-transduced cells show no killing of any targets (solid lines CD70-specific T cells, dashed lines non-transduced T cells).

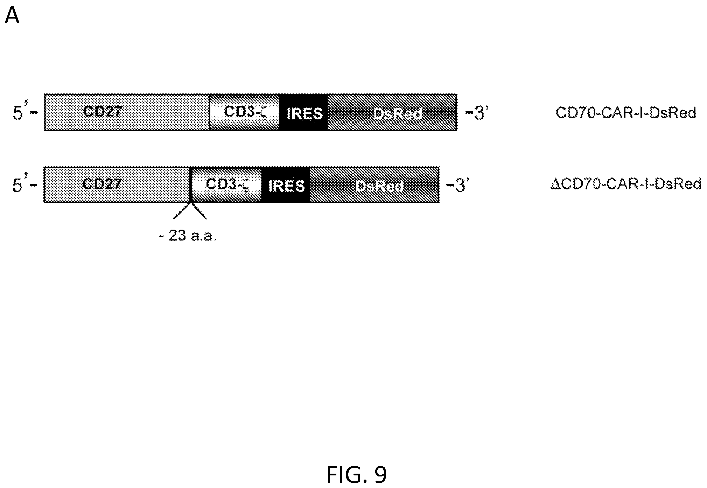

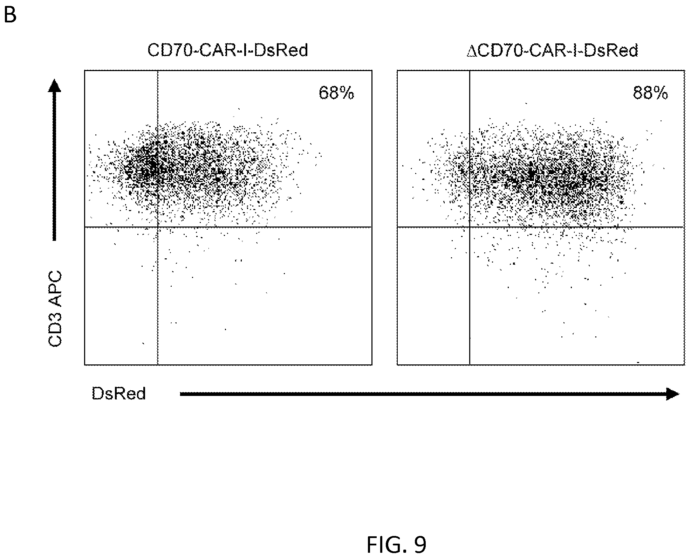

Figure 9. Generation of CD70-CAR and ΔCD70-CAR DsRedexpressing T cells. (A) The CD70-CAR expression cassette was modified to include DsRed for detection and selection of transduced T cells. ΔCD70-CAR was generated by PCR deletion of 23 amino acids in the CD27 endodomain and cloned into the DsRed expression cassette. (B) Transduction efficiency was comparable between T cells expressing CD70-CAR-I-DsRed or ΔCD70-CAR-IDsRed.

### DETAILED DESCRIPTION OF THE INVENTION

As used herein, the use of the word "a" or "an" when used in conjunction with the term "comprising" in the claims and/or the specification may mean "one," but it is also consistent with the meaning of "one or more," "at least one," and "one or more than one." Some embodiments of the invention may consist of or consist essentially of one or more elements, method steps, and/or methods of the invention. It is contemplated that any method or composition described herein can be implemented with respect to any other method or composition described herein.

Targeting CD70-positive malignancies with CD70-specific monoclonal antibodies has shown promise in preclinical animal models (McEarchern *et al*., 2008; Israel *et al*., 2005; McEarchern *et al*., 2007) and the inventors now evaluated whether T cells can be redirected to CD70 by forced expression of the appropriate CAR. Since CARs consist of an extracellular antigen recognition domain derived from murine monoclonal antibodies they may induce human antimouse antibody (HAMA) upon infusion unless fully humanized. (Miotti *et al*., 1999; Kershaw *et al*., 2006) One potential strategy to overcome this limitation is to engineer the antigen recognition domain using endogenous protein ligands or receptors rather than monoclonal antibodies. (Kahlon *et al*., 2004; Zhang *et al*., 2006) To target CD70 with T cells we took advantage of the physiological CD70/CD27 interaction and generated a CD70-specific CAR, which consists of full-length CD27 as the antigen recognition domain fused to the intracellular domain of the CD3- ζ chain. Engagement of chimeric CD27- ζ by tumor targets expressing the CD70 ligand resulted in T-cell activation and CD27 costimulation, which was dependent on the presence of the TRAF2 binding site within the cytoplasmic tail of CD27. CD70-specific T cells killed CD70-positive tumor cell lines as well as primary tumors and had antitumor activity in a murine SCID xenograft model.

### I. Embodiments of Chimeric Receptors of the Invention and Uses Thereof

In embodiments of the invention, there are chimeric receptors that encode a receptor of CD70 and an intracellular signaling domain. In specific aspects, the CD70 receptor is a polypeptide that recognizes the CD70 antigen. In specific embodiments, the receptor of CD70 is CD27.

Although in particular embodiments any suitable intracellular domain is employed in the chimeric receptors of the invention, in specific embodiments it is part or all of the zeta chain of CD3. In specific embodiments, intracellular receptor signaling domains are those of the T cell antigen receptor complex, such as the zeta chain of CD3, also Fcγ RIII costimulatory signaling domains, CD28, DAP10, CD2, alone or in a series with CD3zeta, for example. In specific embodiments, the intracellular domain (which may be referred to as the cytoplasmic domain) comprises part or all of one or more of TCR Zeta chain, CD28, OX40/CD134, 4-1BB/CD137, FcεRIγ, ICOS/CD278, ILRB/CD122, IL-2RG/CD132, and CD40. One or multiple cytoplasmic domains may be employed, as so-called third generation CARs have at least 2 or 3 signaling domains fused together for additive or synergistic effect, for example.

An immunoreceptor according to the present invention can be produced by any means known in the art, though preferably it is produced using recombinant DNA techniques. A nucleic acid sequence encoding the several regions of the chimeric receptor can prepared and assembled into a complete coding sequence by standard techniques of molecular cloning (genomic library screening, PCR, primer-assisted ligation, site-directed mutagenesis, *etc.*). The resulting coding region is preferably inserted into an expression vector and used to transform a suitable expression host cell line, preferably a T lymphocyte cell line, and most preferably an autologous T lymphocyte cell line, a third party derived T cell line/clone, a transformed humor or xerogenic immunologic effector cell line, for expression of the immunoreceptor. NK cells, macrophages, neutrophils, LAK cells, LIK cells, and stem cells that differentiate into these cells, can also be used. In a preferred embodiment, lymphocytes are obtained from a patient by leukopharesis, and the autologous T cells are transduced to express the zetakine and administered back to the individual by any clinically acceptable means, to achieve anti-cancer therapy.

Suitable doses for a therapeutic effect would be between about 10⁶ and about 10⁹ cells per dose, preferably in a series of dosing cycles. A preferred dosing regimen consists of four one-week dosing cycles of escalating doses, starting at about 10⁷ cells on Day 0, increasing incrementally up to a target dose of about 10⁸ cells by Day 5. Suitable modes of administration include intravenous, subcutaneous, intracavitary (for example by reservoir-access device), intraperitoneal, and direct injection into a tumor mass.

As used herein, a nucleic acid construct or nucleic acid sequence is intended to mean a DNA molecule that can be transformed or introduced into a T cell and be transcribed and translated to produce a product (*e.g.,* a chimeric receptor). By example only, GenBank® Accession No. NM_001242 provides a nucleotide sequence for CD27, and this is incorporated by reference herein. Besides CD27, the CD27-ζ molecule contains the signaling domain of the CD3- ζ chain (GenBank® Accession NP_000725.1 and NP_932170.1).

In the nucleic acid construct employed in the present invention, the promoter is operably linked to the nucleic acid sequence encoding the chimeric receptor of the present invention, *i.e.,* they are positioned so as to promote transcription of the messenger RNA from the DNA encoding the chimeric receptor. The promoter can be of genomic origin or synthetically generated. A variety of promoters for use in T cells are well-known in the art (*e.g.,* the CD4 promoter disclosed by Marodon, et al. (2003) Blood 101(9):3416-23). The promoter can be constitutive or inducible, where induction is associated with the specific cell type or a specific level of maturation, for example. Alternatively, a number of well-known viral promoters are also suitable. Promoters of interest include the β-actin promoter, SV40 early and late promoters, immunoglobulin promoter, human cytomegalovirus promoter, retrovirus promoter, and the Friend spleen focus-forming virus promoter. The promoters may or may not be associated with enhancers, wherein the enhancers may be naturally associated with the particular promoter or associated with a different promoter.

The sequence of the open reading frame encoding the chimeric receptor can be obtained from a genomic DNA source, a cDNA source, or can be synthesized (*e.g., via* PCR), or combinations thereof. Depending upon the size of the genomic DNA and the number of introns, it may be desirable to use cDNA or a combination thereof as it is found that introns stabilize the mRNA or provide T cell-specific expression (Barthel and Goldfeld (2003) J. Immunol. 171(7):3612-9). Also, it may be further advantageous to use endogenous or exogenous non-coding regions to stabilize the mRNA.

For expression of a chimeric receptor of the present invention, the naturally occurring or endogenous transcriptional initiation region of the nucleic acid sequence encoding N-terminal component of the chimeric receptor can be used to generate the chimeric receptor in the target host. Alternatively, an exogenous transcriptional initiation region can be used that allows for constitutive or inducible expression, wherein expression can be controlled depending upon the target host, the level of expression desired, the nature of the target host, and the like.

Likewise, a signal sequence directing the chimeric receptor to the surface membrane can be the endogenous signal sequence of N-terminal component of the chimeric receptor. Optionally, in some instances, it may be desirable to exchange this sequence for a different signal sequence. However, the signal sequence selected should be compatible with the secretory pathway of T cells so that the chimeric receptor is presented on the surface of the T cell.

Similarly, a termination region may be provided by the naturally occurring or endogenous transcriptional termination region of the nucleic acid sequence encoding the C-terminal component of the chimeric receptor. Alternatively, the termination region may be derived from a different source. For the most part, the source of the termination region is generally not considered to be critical to the expression of a recombinant protein and a wide variety of termination regions can be employed without adversely affecting expression.

As will be appreciated by one of skill in the art, in some instances, a few amino acids at the ends of the CD27 can be deleted, usually not more than 10, more usually not more than 5 residues, for example. Also, it may be desirable to introduce a small number of amino acids at the borders, usually not more than 10, more usually not more than 5 residues. The deletion or insertion of amino acids may be as a result of the needs of the construction, providing for convenient restriction sites, ease of manipulation, improvement in levels of expression, or the like. In addition, the substitute of one or more amino acids with a different amino acid can occur for similar reasons, usually not substituting more than about five amino acids in any one domain.

The chimeric construct that encodes the chimeric receptor according to the invention can be prepared in conventional ways. Because, for the most part, natural sequences may be employed, the natural genes may be isolated and manipulated, as appropriate, so as to allow for the proper joining of the various components. Thus, the nucleic acid sequences encoding for the N-terminal and C-terminal proteins of the chimeric receptor can be isolated by employing the polymerase chain reaction (PCR), using appropriate primers that result in deletion of the undesired portions of the gene. Alternatively, restriction digests of cloned genes can be used to generate the chimeric construct. In either case, the sequences can be selected to provide for restriction sites which are blunt-ended, or have complementary overlaps.

The various manipulations for preparing the chimeric construct can be carried out *in vitro* and in particular embodiments the chimeric construct is introduced into vectors for cloning and expression in an appropriate host using standard transformation or transfection methods. Thus, after each manipulation, the resulting construct from joining of the DNA sequences is cloned, the vector isolated, and the sequence screened to ensure that the sequence encodes the desired chimeric receptor. The sequence can be screened by restriction analysis, sequencing, or the like.

The chimeric constructs of the present invention find application in subjects having or suspected of having cancer by reducing the size of a tumor or preventing the growth or re-growth of a tumor in these subjects. Accordingly, the present invention further relates to a method for reducing growth or preventing tumor formation in a subject by introducing a chimeric construct of the present invention into an isolated T cell of the subject and reintroducing into the subject the transformed T cell, thereby effecting anti-tumor responses to reduce or eliminate tumors in the subject. Suitable T cells that can be used include, cytotoxic lymphocytes (CTL), tumor-infiltrating-lymphocytes (TIL) or other cells which are capable of killing target cells when activated. As is well-known to one of skill in the art, various methods are readily available for isolating these cells from a subject. For example, using cell surface marker expression or using commercially available kits (*e.g.,* ISOCELL™ from Pierce, Rockford, Ill.).

It is contemplated that the chimeric construct can be introduced into the subject's own T cells as naked DNA or in a suitable vector. Methods of stably transfecting T cells by electroporation using naked DNA are known in the art. See, *e.g.,* U.S. Pat. No. 6,410,319. Naked DNA generally refers to the DNA encoding a chimeric receptor of the present invention contained in a plasmid expression vector in proper orientation for expression. Advantageously, the use of naked DNA reduces the time required to produce T cells expressing the chimeric receptor of the present invention.

Alternatively, a viral vector (*e.g.,* a retroviral vector, adenoviral vector, adeno-associated viral vector, or lentiviral vector) can be used to introduce the chimeric construct into T cells. Suitable vectors for use in accordance with the method of the present invention are non-replicating in the subject's T cells. A large number of vectors are known that are based on viruses, where the copy number of the virus maintained in the cell is low enough to maintain the viability of the cell. Illustrative vectors include the pFB-neo vectors (STRATAGENE®) disclosed herein as well as vectors based on HIV, SV40, EBV, HSV or BPV.

Once it is established that the transfected or transduced T cell is capable of expressing the chimeric receptor as a surface membrane protein with the desired regulation and at a desired level, it can be determined whether the chimeric receptor is functional in the host cell to provide for the desired signal induction. Subsequently, the transduced T cells are reintroduced or administered to the subject to activate anti-tumor responses in the subject. To facilitate administration, the transduced T cells according to the invention can be made into a pharmaceutical composition or made implant appropriate for administration *in vivo,* with appropriate carriers or diluents, which further can be pharmaceutically acceptable. The means of making such a composition or an implant have been described in the art (see, for instance, Remington's Pharmaceutical Sciences, 16th Ed., Mack, ed. (1980)). Where appropriate, the transduced T cells can be formulated into a preparation in semisolid or liquid form, such as a capsule, solution, injection, inhalant, or aerosol, in the usual ways for their respective route of administration. Means known in the art can be utilized to prevent or minimize release and absorption of the composition until it reaches the target tissue or organ, or to ensure timed-release of the composition. Desirably, however, a pharmaceutically acceptable form is employed which does not ineffectuate the cells expressing the chimeric receptor. Thus, desirably the transduced T cells can be made into a pharmaceutical composition containing a balanced salt solution, preferably Hanks' balanced salt solution, or normal saline.

A pharmaceutical composition of the present invention can be used alone or in combination with other well-established agents useful for treating cancer. Whether delivered alone or in combination with other agents, the pharmaceutical composition of the present invention can be delivered *via* various routes and to various sites in a mammalian, particularly human, body to achieve a particular effect. One skilled in the art will recognize that, although more than one route can be used for administration, a particular route can provide a more immediate and more effective reaction than another route. For example, intradermal delivery may be advantageously used over inhalation for the treatment of melanoma. Local or systemic delivery can be accomplished by administration comprising application or instillation of the formulation into body cavities, inhalation or insufflation of an aerosol, or by parenteral introduction, comprising intramuscular, intravenous, intraportal, intrahepatic, peritoneal, subcutaneous, or intradermal administration.

A composition of the present invention can be provided in unit dosage form wherein each dosage unit, *e.g.,* an injection, contains a predetermined amount of the composition, alone or in appropriate combination with other active agents. The term unit dosage form as used herein refers to physically discrete units suitable as unitary dosages for human and animal subjects, each unit containing a predetermined quantity of the composition of the present invention, alone or in combination with other active agents, calculated in an amount sufficient to produce the desired effect, in association with a pharmaceutically acceptable diluent, carrier, or vehicle, where appropriate. The specifications for the novel unit dosage forms of the present invention depend on the particular pharmacodynamics associated with the pharmaceutical composition in the particular subject.

Desirably an effective amount or sufficient number of the isolated transduced T cells is present in the composition and introduced into the subject such that long-term, specific, anti-tumor responses are established to reduce the size of a tumor or eliminate tumor growth or regrowth than would otherwise result in the absence of such treatment. Desirably, the amount of transduced T cells reintroduced into the subject causes a 10%, 20%, 30%, 40%, 50%, 60%, 70%, 80%, 90%, 95%, 98%, or 100% decrease in tumor size when compared to otherwise same conditions wherein the transduced T cells are not present.

Accordingly, the amount of transduced T cells administered should take into account the route of administration and should be such that a sufficient number of the transduced T cells will be introduced so as to achieve the desired therapeutic response. Furthermore, the amounts of each active agent included in the compositions described herein (*e.g.,* the amount per each cell to be contacted or the amount per certain body weight) can vary in different applications. In general, the concentration of transduced T cells desirably should be sufficient to provide in the subject being treated at least from about 1×10⁶ to about 1×10⁹ transduced T cells, even more desirably, from about 1×10⁷ to about 5×10⁸ transduced T cells, although any suitable amount can be utilized either above, *e.g.,* greater than 5×10⁸ cells, or below, *e.g*., less than 1×10⁷ cells. The dosing schedule can be based on well-established cell-based therapies (see, *e.g.,* Topalian and Rosenberg (1987) Acta Haematol. 78 Suppl 1:75-6; U.S. Pat. No. 4,690,915) or an alternate continuous infusion strategy can be employed.

These values provide general guidance of the range of transduced T cells to be utilized by the practitioner upon optimizing the method of the present invention for practice of the invention. The recitation herein of such ranges by no means precludes the use of a higher or lower amount of a component, as might be warranted in a particular application. For example, the actual dose and schedule can vary depending on whether the compositions are administered in combination with other pharmaceutical compositions, or depending on interindividual differences in pharmacokinetics, drug disposition, and metabolism. One skilled in the art readily can make any necessary adjustments in accordance with the exigencies of the particular situation.

### II. Embodiments of Kits of the Invention

Any of the compositions described herein may be comprised in a kit. In a non-limiting example, a chimeric receptor expression construct, one or more reagents to generate a chimeric receptor expression construct, cells for transfection of the expression construct, and/or one or more instruments to obtain autologous cells for transfection of the expression construct (such an instrument may be a syringe, pipette, forceps, and/or any such medically approved apparatus).

The kits may comprise one or more suitably aliquoted compositions of the present invention or reagents to generate compositions of the invention. The components of the kits may be packaged either in aqueous media or in lyophilized form. The container means of the kits may include at least one vial, test tube, flask, bottle, syringe or other container means, into which a component may be placed, and preferably, suitably aliquoted. Where there are more than one component in the kit, the kit also will generally contain a second, third or other additional container into which the additional components may be separately placed. However, various combinations of components may be comprised in a vial. The kits of the present invention also will typically include a means for containing the chimeric receptor construct and any other reagent containers in close confinement for commercial sale. Such containers may include injection or blow molded plastic containers into which the desired vials are retained, for example.

### EXAMPLES

The following examples are included to demonstrate some embodiments of the invention. It should be appreciated by those of skill in the art that the techniques disclosed in the examples which follow represent techniques discovered by the inventor to function well in the practice of the invention, and thus can be considered to constitute some modes for its practice. However, those of skill in the art should, in light of the present disclosure, appreciate that many changes can be made in the specific embodiments which are disclosed and still obtain a like or similar result without departing from the spirit and scope of the invention.

T-cell therapy with genetically modified T cells targeting CD19 or CD20 holds promise for the immunotherapy of hematological malignancies. These targets, however, are only present on B-cell derived malignancies and because they are broadly expressed in the hematopoietic system, their targeting may have unwanted consequences. To expand T-cell therapies to hematologic malignancies that are not B cell derived, the inventors determined whether T cells can be redirected to CD70, an antigen expressed by limited subsets of normal lymphocytes and dendritic cells, but aberrantly expressed by a broad range of hematological malignancies and some solid tumors. To generate CD70-specific T cells the inventors constructed a chimeric antigen receptor (CAR) comprising the CD70 receptor (CD27) fused to the CD3-ζ chain. Stimulation of T cells expressing CD70-specific CARs resulted in CD27 costimulation and recognition of CD70-positive tumor cell lines and primary tumor cells, as shown by IFN-γ and IL-2 secretion and by tumor cell killing. Adoptively transferred CD70-specific T cells induced sustained regression of established murine xenografts. Therefore, CD70-specific T cells are a useful immunotherapeutic approach for CD70-positive malignancies.

### EXAMPLE 1

### EXEMPLARY MATERIALS AND METHODS

### Cell lines and tumor cells

Protocols to obtain blood samples or primary tumor cells were approved by the Baylor College of Medicine Institutional Review Board (IRB). The cell lines Daudi, CCL-120, U266, and K562 were obtained from the American Type Culture Collection (ATCC, Rockville, MD, USA). K562 cells expressing CD70 (K562.70) were generated by transducing K562 cells with a self-inactivating lentiviral vector encoding human CD70 and GFP. L1236 was obtained from DSMZ (Braunschweig, Germany). SNK6 and SNT16 were kindly provided by Dr. Norio Shimizu (Tokyo Medical and Dental University, Japan). (Nagata *et al*., 2001) Primary B-cell non-Hodgkin lymphomas, which had been cryopreserved without *in vitro* culture were provided by Dr. Stephen Ansell (Mayo Clinic, Rochester, MN, USA).

### Generation of the CD70-specific CAR construct

Full-length human CD27 (CD70 receptor) was fused in frame to the signaling domain (amino acids 52-164) of the T-cell receptor ζ-chain (TCR-ζ) using overlap polymerase chain reaction (PCR); pORF.CD27 (Invitrogen, Carlsbad, CA) and pSFG.FRP5.ζ (Ahmed *et al*., 2007) served as PCR templates. Primers were modified to create 5'-NcoI and 3'-SphI restriction sites and the *CD27 TCR-ζ* fusion gene (CD70-CAR) was subcloned into the SFG retroviral vector. To facilitate unequivocal detection of transduced T cells, an internal ribosomal entry sequence (IRES) truncated CD19 (tCD19) (Tey *et al*., 2007) expression cassette (IRES-tCD19) was created by overlap PCR and subcloned 3' of the *CD27 TCR-ζ* fusion gene into 5'-SphI and 3'-AccIII restriction sites of the SFG retroviral vector (pSFG.CD70-CAR-IRES-tCD19; Figure 1A). In addition, a retroviral vector was created containing a CD70-CAR-IRES-DsRed expression cassette or ΔCD70-CAR-IRES-DsRed expression cassette in which the 23 amino acid TRAF2 binding site of CD27 was deleted (residues 238-260 (Yamamoto *et al*., 1998); Figure 8).

### Retrovirus production and transduction of T-lymphocytes

RD114 pseudotyped retroviral particles were generated by transient transfection of 293T cells with the CD70-CAR SFG retroviral vector, Peg-Pam-e plasmid containing the sequence for MoMLV gag-pol, and the RDF plasmid containing the RD114 envelope (Kelly *et al*., 2000), using GeneJuice transfection reagent (Novagen, San Diego, CA). (Vera *et al*., 2006) Supernatant containing the retrovirus was collected 48-72 hours later. For retroviral transduction, non-tissue culture treated 24-well plates were treated overnight with OKT3 (Ortho Biotech, Bridgewater, NJ) and CD28 (Becton Dickinson, Mountain View, CA) antibodies. The following day, 0.5 x 10⁶ peripheral blood mononuclear cells (PBMCs) were added to each well and cultured in RPMI 1640 complete media (Gibco-BRL, Gaithersburg, MD) containing 10% heat inactivated fetal calf serum (FCS) and 1% GlutaMax™(Gibco-BRL). Recombinant human interleukin-2 (rhIL-2; 200 U/mL; Proleukin; Chiron, Emeryville, CA) was added to cultures on day 3. Viral supernatant was added to 24-well plates which were pre-treated with RetroNectin® (Takara Shuzo, Otsu, Japan) and the cultured OKT3/CD28 stimulated cells were added to each well (5 x 10⁵ cells/well). Cells were spun and incubated at 37°C in 5% CO₂. CAR expression on T cells was measured 72 hours later and the cells were maintained in culture in complete media with the addition of rhIL-2 (50-100 U/mL) every 3 days. Non-transduced T cells, used as controls, were activated with OKT3/CD28 and expanded in the presence of 50-100 units IL-2 per mL for 10-15 days prior to use.

### Flow cytometry

A FACS Calibur instrument (BD Biosciences) was used to acquire immunofluorescence data, which were analyzed with FCS Express software Version 3 (De Novo Software, Los Angeles, CA). All antibodies for surface staining were purchased from BD Biosciences. Isotype controls were immunoglobulin G1- fluorescein isothiocyanate (IgG1-FITC), IgG1-phycoerythrin (IgG1-PE), IgG1- peridinin chlorophyll protein (IgG1-PerCP), and IgG1-allophycocyanin (IgG1- APC). Forward and side scatter gating were used to discriminate live cells from dead cells. CD70-CAR expression was analyzed on 293 T cells using CD27-FITC, CD19-PE and on human CD3/CD28 stimulated T cells using CD19-PE, CD3-FITC, CD4-PerCP, and CD8-APC. CD70 expression on tumor cells was determined using CD70-PE. For Intracellular staining, cells were fixed with 4% paraformaldehyde (BD) and permeabilized with 1% saponin (Sigma). A mouse monoclonal antibody to Bcl-xl (Santa Cruz Biotechnology, Inc., Santa Cruz, CA) was used for primary staining and goat anti-mouse APC (GAM-APC; BD) was used for secondary staining. Isotype controls were cells incubated with GAMAPC alone.

### Analysis of cytokine production

CD70-specific or non-transduced T cells from healthy donors were co-cultured with CD70-positive cell lines or primary CD70-positive lymphomas at a 2:1 effector to target ratio in a 48-well plate. After 24 hours of incubation, culture supernatants were harvested and the inventors measured IFN-γ and IL-2 by ELISA as per the manufacturer's instructions (R&D Systems, Minneapolis, MN).

### IFN-γ ELISPOT assay

The inventors used ELISPOT assays, as described previously, (Gottschalk *et al*., 2003) to determine the frequency of IFN-γ -secreting T cells. CD70-CAR or nontransduced T cells were plated at 1 x 10⁵ and incubated for 18 hours with the appropriate stimulus. Plates were then developed, dried overnight, and sent to ZellNet Consulting (New York, NY) for quantification.

### Co-Immunoprecipitation

293T cells stably expressing CD70-CAR or ΔCD70-CAR were generated by retroviral transduction. Cells expressing CARs were transfected with 2 µg of FLAG-tagged TRAF2, kindly provided by Dr. Jinhua Yang (Baylor College of Medicine), using GeneJuice transfection reagent (Novagen, San Diego, CA). Twenty-four hours after transfection the cells were co-cultured with K562.70 cells at a ratio of 1:1 to cross-link the receptor. After 12 hours, cells were washed with ice cold PBS (Sigma, St. Louis, MO) and the non-adherent K562.70 cells were aspirated from the culture. The remaining 293T cells were lysed and proteins precipitated with anti-FLAG® M2 antibody (Sigma) using µMACS™ Protein G MicroBeads and a µColumn (Miltenyi Biotec Inc., Auburn, CA). The immunoprecipitate was separated by SDS-PAGE and blotted with a CD3-ζ antibody (Santa Cruz Biotechnology).

### Chromium-release assay

Standard chromium-release assays were performed in triplicates as previously described. (Gottschalk *et al*., 2003) Briefly, 1x10⁶ target cells were labeled with 0.1 mCi (3.7MBq) ⁵¹Cr and mixed with decreasing numbers of effector cells to give effector to target ratios of 40:1, 20:1, 10:1 and 5:1. Target cells incubated in complete medium alone or in 1% Triton X-100 were used to determine spontaneous and maximum ⁵¹Cr release, respectively. After 4 hours supernatants were collected and radioactivity was measured in a gamma counter (Cobra Quantum; PerkinElmer; Wellesley; MA). The mean percentage of specific lysis of triplicate wells was calculated according to the following formula: [test release - spontaneous release] / [maximal release - spontaneous release] x 100.

### CFSE proliferation and long-term killing assay

To measure T-cell proliferation and long-term killing the inventors incubated 1 x 10⁷ T cells for 10 minutes at room temperature with 1.5 µM carboxyfluorescein diacetate succinimidyl ester (CFSE; Molecular Probes, Inc., Eugene, OR). The inventors cultured CFSE-labeled T cells in the absence of exogenous IL-2 with the appropriate CD70-positive or CD70-negative tumor cells at a 2:1 effector:target ratio. After 5-7 days of co-culture cells were collected, stained with CD3, and analyzed for CFSE dilution by FACS analysis. Positive and negative controls for proliferation experiments were T cells cultured in the presence of 100 U/ml rhIL-2 and T cells alone with no cytokine, respectively. For long-term killing experiments, FACS analysis was performed using forward and side scatter gating to determine viable cells, while CFSE staining and CD3-positivity was used to distinguish CD70- specific or non-transduced T cells from CD3-negative, unlabeled tumor cells.

### Xenograft model and bioluminescence imaging

All animal experiments were conducted under a protocol approved by the Baylor College of Medicine Institutional Animal Care and Use Committee. To assess the antitumor effect of CD70-specific T cells *in vivo,* the inventors used 2 SCID mouse models and an IVIS (Caliper Life Sciences) *in vivo* imaging system. (Ahmed *et al*., 2007) Eight- to 10-week-old SCID mice (IcrTac:ICR-*Prkdc^{scid}*; Taconic) were sublethally irradiated (2.5 Gy) and 2 days later, 5 x 10⁵ Daudi cells expressing an enhanced GFP (eGFP)-firefly luciferase (eGFP-FFLuc) fusion gene, suspended in Matrigel (BD Biosciences) were injected IP. To monitor tumor growth, isoflurane-anesthetized animals were injected IP with D-luciferin (150 mg/kg), and a bioluminescence image was obtained and analyzed after 10 minutes using Living Image software Version 4.0 (Caliper Life Sciences). A constant region of interest was drawn over the tumor region and the intensity of the signal measured as total photons per second per square centimeter per steradian (p/s/cm2/sr) was obtained. After 10 days, when the tumor signal was consistently increasing, mice were treated with CD70-specific or nontransduced T cells. Three IP injections of 1 x 10⁷ T cells were given on days 10, 11, and 17, followed by 1500 U of rhIL-2 (R&D Systems) also given IP. Mice were imaged before each T-cell injection and 3 times weekly thereafter. The inventors used a Raji SCID xenograft to evaluate the antitumor activity of CD70-specific T cells in a systemic non-Hodgkin lymphoma model.(Brentjens *et al*., 2003; Cheadle *et al*., 2008; Tammana *et al*., 2010) Briefly, 2 x 10⁵ Raji.FFluc cells were injected IV into sublethally irradiated (2.5 Gy) SCID mice, which were treated 4 days later by IV administration of 1 x 10⁷ CD70-specific or nontransduced T cells. The inventors gave 3 doses of T cells (day 4, 5, and 11) with 1500 U of rhIL-2. The inventors quantified metastatic tumors using bioluminescence imaging. For survival analysis, mice were euthanized at the first sign of hind-limb paralysis, identified as one or both limbs dragging while walking.

### Statistical analysis

Comparisons of IFN-γ and IL-2 secretion between CD70-specific and nontransduced T cells were performed using the Wilcoxon signed-rank test. Tumor volume data were log transformed and changes from initial T-cell injection to post-treatment measurements were calculated. Pairwise comparisons were employed to identify any statistically significant difference in light intensity between the two T-cell groups. A p-value less than 0.05 was considered statistically significant. The survival curves were constructed using the Kaplan-Meier method and compared using the weighted long-range test.

### EXAMPLE 2

### GENERATION OF CD70-SPECIFIC T CELLS

The inventors constructed an SFG retroviral vector that encoded the CD70 receptor, CD27, fused to the signaling domain of the T-cell receptor ζ chain (CD70-CAR). Because most naive and memory T cells endogenously express low levels of CD27, an IRES-tCD19 expression cassette was also included in the retroviral vector to allow for unequivocal detection of transduced cells (Figure 1A). CD27 and tCD19 displayed a linear co-expression pattern indicating that tCD19 is a suitable marker for CD70-CAR expression (Figure 1B). CD3/CD28 activated T cells were transduced with RD114-pseudotyped retroviral particles encoding CD70-CAR-IRES-tCD19 and 10 to 14 days post transduction the expression of tCD19 was determined by FACS analysis. A mean of 45% (+/- 6; n=5) T cells expressed tCD19, and both CD4- and CD8-positive cells were transduced (Figure 1C-D).

### EXAMPLE 3

### CD70-SPECIFIC T CELLS SECRETE IMMUNOSTIMULATORY CYTOKINES AND PROLIFERATE AFTER EXPOSURE TO CD70-POSITIVE TUMOR CELLS

To detect recognition of CD70 by transgenic T cells, the inventors initially used CD70-negative K562 cells and CD70-transgenic K562 cells (Figure 2). CD70-specific T cells and non-transduced T cells of 3 donors were stimulated with K562 or K562.CD70, and after 48 hours we measured IFN-γ and IL-2 release (Figure 3A,B). CD70-specific T cells produced significant amounts of IFN-γ (p=0.03) and IL-2 (p=0.02) after exposure to K562.CD70 as compared to non-transduced T cells. In addition, CD70-negative K562 cells did not activate CD70-specific T cells, indicating that cytokine production requires both the expression of CD70 on target cells and the presence of the CD70-CAR on T cells. There was a similar outcome when the inventors compared T-cell proliferation in each of these culture combinations (Figure 3C).

The inventors confirmed the above findings by using tumor cells in which CD70 expression was naturally present but at variable levels. They used a panel of CD70-positive tumor cell lines representing Non-Hodgkin's lymphoma (Daudi, SNK6, SNT16), Hodgkin's lymphoma (L1236), leukemia (CCL-120) and multiple myeloma (U266; Figure 2). CD70-specific T cells secreted significantly more IFN-γ (p<0.0001) and IL-2 (p<0.0001) than non-transduced T cells (Figure 3A,B). T-cell proliferation was dependent on the expression of CD70 on target cells, and CD70^{dim} tumor cells (Daudi) induced less T-cell proliferation than CD70^{bright} tumor cells. In addition, the inventors observed proliferation of nontransduced T cells after stimulation with SNT16 cells, which the inventors attributed to low levels of IL-2 secretion by the SNT16 cells (10-50 pg/mL) and to their robust ability to co-stimulate, as judged by their ability to induce IL-2 production of CD70-specific T cells (Figure 1B). The expression of CD70 was low to absent on peripheral blood B and T cells from healthy donors (Figure 2). Accordingly, the inventors could not detect IFN-γ or IL-2 production of CD70-specific T cells after coculture with primary B or T cells. To confirm that CD70-specific T cells are not stimulated by B or T cells, the inventors used an IFN-γ ELISPOT assay, which showed no activation of CD70-specific T cells after coculture with primary B or T cells (Figure 8A).

### EXAMPLE 4

### CD70-SPECIFIC T CELLS KILL CD70-POSITIVE TUMOR CELLS BUT NOT CD70-NEGATIVE CELLS

The inventors next measured the killing of CD70-positive targets by CD70-specific T cells in both a standard 4 h ⁵¹Cr-release assay and a 5 to 7 day coculture assay. In the 4 h ⁵¹Cr-release assay, CD70-specific T cells killed CD70-positive target cells (K562.70, Daudi, U266, SNK6, SNK16) but not CD70-negative cells (K562). Nontransduced T cells showed no killing confirming CD70-specificity (Figure 4A,B). For the coculture assays, CD70-specific or non-transduced T cells were labeled with CFSE and added to unlabeled tumor cells at a ratio of 2:1. After 5 to 7 days, tumor cells were enumerated by FACS analysis of the CD3-/CFSE-negative fraction; (Figure 4C). CD70-specific T cells eliminated all four CD70-positive lines tested (Daudi, U266, SNK6, SNK16), while control T cells could not (Figure 4D). Whereas T cells stimulated with CD3/CD28 were not killed by CD70-specific T cells, B-cell blasts activated "super-physiologically" with the CD40 ligand on MRC5 cells were susceptible to CD70-specific T-cell killing (Figure 8B).

### EXAMPLE 5

### CD27 COSTIMULATION IS IMPORTANT FOR T-CELL SURVIVAL POST CD70-SPECIFIC STIMULATION

To determine the role of the 23 amino acid co stimulatory domain of CD27 located within the endodomain of the CD70-CAR (Figure 1A), the inventors generated a CD70-CAR with a deleted CD27 costimulatory domain (ΔCD70-CAR). Functional absence of the costimulatory domain was confirmed by the inability of ΔCD70- CAR to bind to TRAF2, the key adaptor protein mediating CD27 signaling (Figure 5A). T cells were transduced with retroviral vectors encoding CD70-CAR-I-dsRed or ΔCD70-CAR-I-dsRed (Figure 9A). Transduction efficiencies of both constructs were similar as judged by dsRed expression (65 to 90%; Figure 9B), and in cytotoxicity assays CD70-CAR and ΔCD70- CAR expressing T cells killed CD70-positive targets with the same efficiency (Figure 5B). To assess the contribution of CD27 costimulation to T-cell activation, the inventors took advantage of autologous fibroblasts, which are devoid of costimulatory molecules and were genetically modified to express CD70 (Fib.CD70). Starting 3 days post T-cell stimulation with Fib.CD70, there were significantly larger "clumps" of activated CD70-CAR T cells in comparison to ΔCD70-CAR T cells (Figure 5C). While there was no difference in T-cell proliferation (Figure 5D) and production of IFN-γ or IL-2, ΔCD70-CAR T-cell viability was significantly reduced in comparison to CD70-CAR T cells (Figure 5D; *P*<0.05). As reported by others, Bcl-xl, an important anti-apoptotic protein, is induced by CD27 signaling. (van Oosterwijk *et al*., 2007) In agreement with this finding CD70-CAR T cells consistently expressed higher levels of Bcl-xl in comparison to ΔCD70-CAR T cells (Figure 5E). These results indicate that the CD27 costimulatory domain located within CD70-CAR provides a costimulatory signal, resulting in enhanced T-cell survival. For all subsequent experiments we therefore used CD70-CAR T cells (CD70-specific T cells).

### EXAMPLE 6

### CD70-SPECIFIC T CELLS RECOGNIZE AND KILL PRIMARY B- AND T-CELL LYMPHOMAS

Having shown that CD70-specific T cells recognize and kill CD70-positive lymphoma cell lines, the inventors next validated the CD70 antigen as a target on primary Band T- cell lymphomas. The inventors co-cultured primary CD70-positive B-cell non- Hodgkin's lymphoma (MF1792, MF1731, MF888) and T-cell acute lymphoblastic leukemia (T007) cells with CD70-specific T cells from a healthy donor for 24 hours, and measured IFN-γ in the supernatants. CD70-specific T cells but not control T cells produced IFN-γ secretion on exposure to CD70+ malignancies. (Figure 6A). In 5 day coculture assays, CD70-specific T cells but not control T cells eliminated primary CD70-positive cells (Figure 6B,C). Hence, CD70-specific T cells recognize and kill primary CD70-positive malignant cells in a CD70- specific manner.

### EXAMPLE 7

### IN VIVO REGRESSION OF ESTABLISHED LYMPHOMA AFTER ADMINISTRATION OF CD70-SPECIFIC T CELLS.

The inventors measured the antitumor activity of CD70-specific T cells in a xenogenic SCID mouse model. The inventors injected 5 x 10⁵ Daudi.FFluc cells i.p. into sublethally irradiated SCID mice and followed tumor growth by serial bioluminescence imaging of mice. After 10 days mice received three injections of 1 x 10⁷ CD70- specific T cells given 1 day and then 1 week apart (injection days 0, 1, and 7; n=10). A second group of tumor-bearing mice was injected with non-transduced T cells. In mice treated with non-transduced T cells, the tumors grew exponentially as judged by bioluminescence imaging (Figure 7A). In contrast, there was a significant difference in tumor burden between CD70-specific and non-transduced T cell groups at day 7 post T-cell injection (p=0.002) (Figure 7B). In 8 of 9 mice with growing tumors, photon emission returned to baseline after CD70-specific T-cell injection, indicating tumor regression that was sustained in 7 mice for > 2 weeks after T-cell transfer.

In a second *in vivo* study, the inventors measured the antitumor activity of CD70-specific T cells using a systemic lymphoma model. The inventors injected 2 x 10⁵ Raji.FFluc cells IV into sublethally irradiated SCID mice. After 4 days, the inventors gave the mice 3 IV injections of 1 x 10⁷ CD70-specific or nontransduced T cells using the same treatment schema described in the previous paragraph. Systemic tumors were enumerated using bioluminescence imaging. At weeks 3 and 4 after tumor cell injection, there was a significantly higher (*P*= .012 and *P* = .10, respectively) tumor burden in mice receiving nontransduced T cells than CD70-specific T cells (Figure 7C). This translated into a significant increase (*P* < .05) in overall survival in mice treated with CD70-specific T cells (Figure 7D).

### EXAMPLE 8

### PRIMARY CD70-POSITIVE T-CELL LYMPHOMA CELLS ASSOCIATED WITH SEVERE CHRONIC ACTIVE EBV INFECTION ARE KILLED BY CD70-SPECIFIC T CELLS

Severe chronic active Epstein-Barr virus infection (CAEBV) is a rare complication of latent EBV infection. It occurs predominately in Japan but several cases have been reported in the western hemisphere (Kimura *et al*., 2003; Cohen *et al*., 2008). In CAEBV natural killer (NK), T cells, or rarely B cells are infected, predisposing patients to life-threatening complications, such as hemophagocytic syndrome and NK- or T-cell lymphoproliferative disease (LPD) (Kimura et al,. 2001; Ishihara *et al*., 1997). The only curative option for CAEBV-associated LPD is currently stem cell transplantation. In this example, the inventors report a patient who developed an aggressive T-cell lymphoma in the setting of CAEBV.

The inventors now demonstrate that CD70 is expressed in primary CAEBV-associated T-cell lymphoma cells, and that these cells are sensitive to killing by CD70-specific T cells, identifying CD70 as a potential immunotherapeutic target for CAEBV-associated T-cell lymphoma.

### EXAMPLE 9

### SIGNIFICANCE OF CERTAIN EMBODIMENTS OF THE INVENTION

The inventors show that CD70, which is aberrantly expressed on several hematologic malignancies and carcinomas, can be targeted by T cells engineered to express CD27 as part of a CAR. T cells expressing a CD70-specific CAR recognized and killed CD70-positive tumor cell lines and primary tumor samples *in vitro* and eliminated human CD70 tumors in a mouse xenograft.

Although present on many leukemias and lymphomas, CD70 is not a lineage-specific marker, and physiologically it is only expressed transiently in subsets of highly activated T, B, and dendritic cells. The CD70 promoter contains transcription factor-binding sites for AP-1, AP-2, Sp1, and NF-κB, and is sensitive to methylation; however, the precise signaling pathways that regulate CD70 expression are poorly understood. (Lu *et al*., 2005) CD70 is up-regulated in human T-lymphotropic virus type 1- and EBV-associated malignancies and Hodgkin lymphomas, likely in association with constitutive NF-κB activation, a pathway that might contribute to regulating CD70 expression. (Nolte *et al*., 2009; Jost *et al*., 2007) The role of aberrant CD70 expression on malignant cells is less well understood than its physiologic contributions, but it may contribute to immune evasion by non-Hodgkin lymphoma.(Yang *et al*., 2007) Others have shown that the CD70/CD27 costimulatory pathway is critical for inducing leukemia-specific T-cell responses.(Glouchkova *et al*., 2009)

The exodomains of most CARs consist of modified monoclonal antibody-binding sites that can be used to prepare antigen-specific T cells that recognize and kill tumor cells in a MHC-nonrestricted fashion. Unless these monoclonal antibody fragments are humanized, they may induce human anti-mouse antibody and/or endogenous T-cell responses that abbreviate the effector function of the infused cells. (Miotti *et al*., 1999; Kahlon *et al*., 2004; Jensen *et al*., 2010) Thus, taking advantage of physiologically occurring receptor-ligand interactions (Kahlon *et al*., 2004; Zhang *et al*., 2006) bypasses this obstacle and should ensure that *in vivo* effector function in human subjects is not interrupted by an unwanted immune response to the transgene. The inventors therefore constructed a CD70-specific CAR by fusing the CD3-ζ chain to the naturally occurring CD70 receptor CD27.

Stimulation of CD70-specific T cells with CD70-positive tumor cells resulted in the secretion of both IFN-γ and IL-2. Whereas triggering of CARs containing only a ζ-signaling domain results in IFN-γ production, IL-2 is generally only secreted in an antigen-dependent manner.(Ahmed *et al*., 2007) Coculture of CD70- specific T cells with CD70-positive tumor cells resulted in the production of 4000-14 000 pg/mL of IFN-γ by CD70-specific T cells, (Ahmed *et al*., 2009) which is within the range reported for other CARexpressing T cells. Because CAR T-cell activation is dependent on the antigen density on target cells,(Weijtens *et al*., 2000) as well as on the presence of costimulatory molecules,(Zhao et al,. 2009) it is not surprising that IFN-γ production varied between individual CD70-positive tumor cell lines. Daudi cells, which induced the lowest level of IFN-γ secretion, had the lowest expression of CD70 as judged by FACS analysis. In addition to IFN-γ production, the inventors observed significant-though variable-secretion of IL-2 after exposure to tumor cells. These differences were independent of tumor CD70 expression levels and did not appear to be dependent on the expression of conventional costimulation molecules, because the inventors observed IL-2 secretion after T-cell stimulation with K562.70 cells, which do not express classic costimulatory molecules such as CD80 and CD86. These cells do, however, express NKG2D ligands, which can provide costimulatory signals by interacting with NKG2D expressed on human CD8-positive T cells. (Maasho *et al*., 2005) Moreover, SNT16 and SNK6 non-Hodgkin lymphoma cells induced high levels of IL-2 production from CD70-specific T cells, an effect consistent with the known high expression of adhesion molecules on EBV-positive, NK/T-cell non-Hodgkin lymphoma cells. (Kanno *et al*., 2008)

CD27 costimulation prevents activation-induced cell death in T cells, in part by up-regulation of Bcl-xl, an antiapoptotic protein.(van Oosterwijk *et al*., 2007) In agreement with this finding, the inventors observed that T cells expressing ΔCD70-CARs with a deleted CD27 costimulatory domain had decreased viability and lower levels of Bcl-xl expression than T cells expressing CD70-CARs with full-length CD27. These data indicate that CD70-CAR T cells may also exhibit prolonged persistence *in vivo.* Interestingly, *in vivo* efficacy data of ex vivo-expanded tumor-infiltrating lymphocytes suggest that the expression of CD27 is correlated with antitumor activity.(Huang *et al*., 2006) One can determine whether CD27 costimulation enhances the persistence of CAR-expressing T cells.

Whereas the inventors observed complete killing of CD70-positive tumor cells in a 5- to 7-day coculture assay (Figure 4C-D), the inventors observed more variable levels of tumor cell killing in a standard 4-hour ⁵¹Cr-release assay (Figure 4B). These differences were most likely T-cell independent, because the kinetics of tumor cell disintegration (chromium release) depends on their intrinsic sensitivity to T cell-derived cytotoxic molecules such as perforin or granzyme B rather than to differences in the effector function of the T cell itself. (Perelson *et al*., 1984)

In embodiments of the invention, CD70-specific T cells expressing CD27-ζ CARs displayed significant *in vivo* antitumor activity in both an IP Daudi and IV Raji model of lymphoma. The observed antitumor activity of CD70-specific T cells in the IP Daudi model was similar to T cells expressing CD19-CARs, as reported previously. (Tammana *et al*., 2010; Kowolik *et al*., 2006; Hoyos *et al*., 2010) Interestingly, sustained antitumor responses, as observed with CD70-specific T cells, were only observed with CD19-specific T cells expressing CARs that contained costimulatory domains. This indicates that CD27- ζ CARs provide costimulatory signals *in vivo,* in specific embodiments, as the inventors have shown in our *in vitro* experiments (Figure 5). The requirement for costimulatory domains for CD19-CARs to kill tumor cells in the IV Raji model is controversial and contradictory. (Brentjens *et al*., 2003; Cheadle *et al*., 2008; Tammana *et al*., 2010) These conflicting results might be explained by differences in the ex vivo preparation of genetically modified T cells, the strain of immunodeficient mice, and/or the particular Raji cell line derivative used for the *in vivo* experiments, in certain aspects.

Because CD70 is physiologically expressed by a subset of immune cells during activation, the targeting of this receptor with CAR T cells might potentially impair cellular immune responses. However, the inventors consider this unlikely because CD70 is only expressed transiently on a small proportion of activated lymphocytes and dendritic cells. In addition, CD27-knockout mice (lacking any CD27/CD70 costimulation) have only subtle changes in their immune systems, with protective primary antigen-specific T-cell responses but a smaller memory T-cell compartment compared with normal mice after pathogen exposure. (Hendriks *et al*., 2000; Nolte *et al*., 2009) These subtle changes are unlikely to be of major relevance in adult human subjects, in whom reactivation of preexisting memory populations is the dominant response to infection. In the studies, CD70-specific T cells showed no reactivity against peripheral blood B and T cells. The inventors also showed that activated T cells are not killed by CD70-specific T cells in cytotoxicity assays (Figure 8B). In contrast, B-cell blasts were susceptible to CD70-specific T-cell killing, but only after activation with the CD40 ligand and after allogeneic feeder cells had induced CD70 expression (Figure 8B). Whereas these results indicate that CD70-specific T cells have the potential to kill activated B cells, the physiologic relevance of this finding remains uncertain because this type of "super-physiologic" B-cell activation, resulting in prolonged CD70 expression, does not occur *in vivo.* Indeed, it has been demonstrated that CD70 is readily expressed on the surface of murine B cells stimulated *in vitro* with CD40 monoclonal antibodies and lipopolysaccharide; however, mice challenged with influenza virus show virtually no surface expression of CD70 on B cells infiltrating the lungs and draining lymph nodes.(Tesselaar *et al*., 2003) Likewise, CD70-expressing B cells are rarely observed in humans, being found on a limited number of germinal center B cells in less than 10% of tonsils examined and on scattered lymphocytes in secondary lymphoid organs and peripheral blood.(Hintzen *et al*., 1994) No side effects have been reported so far in 2 phase 1 clinical studies evaluating the safety and tolerability of CD70 monoclonal antibodies (MDX-1203, NCT00944905; SGN-75, NCT01015911).

In summary, CD70-specific T cells can be readily generated by gene transfer with CARs encoding CD27-ζ, and these cells can kill human tumors *in vitro* and *in vivo.* Adoptive transfer of CD70-redirected T cells may be an attractive immunotherapeutic approach for B or T cell-derived hematologic malignancies and other CD70-positive solid tumors.

### REFERENCES

All patents and publications mentioned in the specifications are indicative of the levels of those skilled in the art to which the invention pertains. All patents and publications are herein incorporated by reference to the same extent as if each individual publication was specifically and individually indicated to be incorporated by reference.

### PATENTS

U.S. Pat. No. 4,690,915
U.S. Pat. No. 6,410,319

### PUBLICATIONS

Agathanggelou A, Niedobitek G, Chen R et al. Expression of immune regulatory molecules in Epstein-Barr virus-associated nasopharyngeal carcinomas with prominent lymphoid stroma. Evidence for a functional interaction between epithelial tumor cells and infiltrating lymphoid cells. Am.J.Pathol. 1995;147:1152-1160.
Ahmed N, Ratnayake M, Savoldo B et al. Regression of experimental medulloblastoma following transfer of HER2-specific T cells. Cancer Res 2007;67:5957-5964.
Baba M, Okamoto M, Hamasaki T et al. Highly enhanced expression of CD70 on human T-lymphotropic virus type 1-carrying T-cell lines and adult T-cell leukemia cells. J Virol. 2008;82:3843-3852.
Bollard CM, Gottschalk S, Leen AM, et al. Complete responses of relapsed lymphoma following genetic modification of tumor-antigen presenting cells and T-lymphocyte transfer. Blood 2007;110:2838-2845.
Bowman MR, Crimmins MA, Yetz-Aldape J et al. The cloning of CD70 and its identification as the ligand for CD27. J Immunol. 1994;152:1756-1761.
Chahlavi A, Rayman P, Richmond AL et al. Glioblastomas induce Tlymphocyte death by two distinct pathways involving gangliosides and CD70. Cancer Res 2005;65:5428-5438.
Cohen JI, Kimura H, Nakamura S, et al. Epstein-Barr virus-associated lymphoproliferative disease in non-immunocompromised hosts: a status report and summary of an international meeting, 8-9 September 2008. Ann Oncol 2009;20:1472-1482.
Cooper LJ, Al Kadhimi Z, Serrano LM et al. Enhanced antilymphoma efficacy of CD19-redirected influenza MP1-specific CTLs by cotransfer of T cells modified to present influenza MP1. Blood 2005;105:1622-1631.
Di Stasi A, De Angelis B, Rooney CM et al. T lymphocytes coexpressing CCR4 and a chimeric antigen receptor targeting CD30 have improved homing and antitumor activity in a Hodgkin tumor model. Blood 2009; 113:6392-6402.
Glouchkova L, Ackermann B, Zibert A et al. The CD70/CD27 pathway is critical for stimulation of an effective cytotoxic T cell response against B cell precursor acute lymphoblastic leukemia. J Immunol. 2009;182:718-725.
Gotoh K, Ito Y, Shibata-Watanabe Y, et al. Clinical and virological characteristics of 15 patients with chronic active Epstein-Barr virus infection treated with hematopoietic stem cell transplantation. Clin Infect Dis 2008;46:1525-1534.
Gottschalk S, Edwards OL, Sili U et al. Generating CTL against the subdominant Epstein-Barr virus LMP1 antigen for the adoptive 29 Immunotherapy of EBV-associated malignancies. Blood 2003;101:1905-1912.
Hendriks J, Gravestein LA, Tesselaar K et al. CD27 is required for generation and long-term maintenance of T cell immunity. Nat.Immunol. 2000;1:433-440.
Hintzen RQ, Lens SM, Beckmann MP et al. Characterization of the human CD27 ligand, a novel member of the TNF gene family. J Immunol. 1994;152:1762-1773.
Hunter ZR, Branagan AR, Santos DD et al. High levels of soluble immunoregulatory receptors in patients with Waldenstrom's macroglobulinemia. Blood 2004;104:4881. 26
Ishihara S, Okada S, Wakiguchi H, et al. Clonal lymphoproliferation following chronic active Epstein-Barr virus infection and hypersensitivity to mosquito bites. Am J Hematol 1997;54:276-281.
Israel BF, Gulley M, Elmore S et al. Anti-CD70 antibodies: a potential treatment for EBV+ CD70-expressing lymphomas. Mol.Cancer Ther. 2005;4:2037-2044.
Jensen MC, Popplewell L, Cooper LJ et al. Anti-Transgene Rejection Responses Contribute to Attenuated Persistence of Adoptively Transferred CD20/CD19-Specific Chimeric Antigen Receptor Re-directed T Cells in Humans. Biol.Blood Marrow Transplant. 2010 30
Jost PJ, Ruland J. Aberrant NF-kappaB signaling in lymphoma: mechanisms, consequences, and therapeutic implications. Blood 2007;109:2700-2707.
June CH. Adoptive T cell therapy for cancer in the clinic. J.Clin.Invest 2007;117:1466-1476.
Junker K, Hindermann W, von Eggeling F et al. CD70: a new tumor specific biomarker for renal cell carcinoma. J Urol. 2005;173:2150-2153.
Kahlon KS, Brown C, Cooper LJ et al. Specific recognition and killing of glioblastoma multiforme by interleukin 13-zetakine redirected cytolytic T cells. Cancer Res 2004;64:9160-9166.
Kawa K, Sawada A, Sato M, et al. Excellent outcome of allogeneic hematopoietic SCT with reduced intensity conditioning for the treatment of chronic active EBV infection. Bone Marrow Transplant 2011;46:77-783.
Kelly PF, Vandergriff J, Nathwani A, Nienhuis AW, Vanin EF. Highly efficient gene transfer into cord blood nonobese diabetic/severe combined immunodeficiency repopulating cells by oncoretroviral vector particles pseudotyped with the feline endogenous retrovirus (RD114) envelope protein. Blood 2000;96:1206-1214.
Kershaw MH, Westwood JA, Parker LL et al. A phase I study on adoptive immunotherapy using gene-modified T cells for ovarian cancer. Clin.Cancer Res. 2006;12:6106-6115.
Kimura H, Morishima T, Kanegane H, et al. Prognostic factors for chronic active Epstein-Barr virus infection. J Infect Dis 2003;187:527-533.
Kimura H, Hoshino Y, Kanegane H, et al. Clinical and virologic characteristics of chronic active Epstein-Barr virus infection. Blood 2001;98:280-286.
Leen AM, Rooney CM, Foster AE. Improving T cell therapy for cancer. Annu.Rev.Immunol. 2007;25:243-265.
Lens SM, Drillenburg P, den Drijver BF et al. Aberrant expression and reverse signalling of CD70 on malignant B cells. Br.J Haematol. 1999;106:491-503.
Lu Q, Wu A, Richardson BC. Demethylation of the same promoter sequence increases CD70 expression in lupus T cells and T cells treated with lupus-inducing drugs. J Immunol. 2005;174:6212-6219.
Maasho K, Opoku-Anane J, Marusina AI, Coligan JE, Borrego F. NKG2D is a costimulatory receptor for human naive CD8+ T cells. J Immunol. 2005;174:4480-4484.
McEarchern JA, Oflazoglu E, Francisco L et al. Engineered anti-CD70 antibody with multiple effector functions exhibits in vitro and in vivo antitumor activities. Blood 2007;109:1185-1192.
McEarchern JA, Smith LM, McDonagh CF et al. Preclinical characterization of SGN-70, a humanized antibody directed against CD70. Clin Cancer Res. 2008;14:7763-7772.
Miotti S, Negri DR, Valota O et al. Level of anti-mouse-antibody response induced by bi-specific monoclonal antibody OC/TR in ovarian-carcinoma patients is associated with longer survival. Int.J Cancer 1999;84:62-68.
Nagata H, Konno A, Kimura N et al. Characterization of novel natural killer (NK)-cell and gammadelta T-cell lines established from primary lesions of 28 nasal T/NK-cell lymphomas associated with the Epstein-Barr virus. Blood 2001;97:708-713.
Nolte MA, van Olffen RW, van Gisbergen KP, van Lier RA. Timing and tuning of CD27-CD70 interactions: the impact of signal strength in setting the balance between adaptive responses and immunopathology. Immunol.Rev. 2009;229:216-231. 27
Ohshima K, Kimura H, Yoshino T, et al. Proposed categorization of pathological states of EBVassociated T/natural killer-cell lymphoproliferative disorder (LPD) in children and young adults: Overlap with chronic active EBV infection and infantile fulminant EBV T-LPD. Pathol Int 2008; 58:209-217.
Ohshima K, Suzumiya J, Sugihara M, et al. Clinicopathological study of severe chronic active Epstein-Barr virus infection that developed in association with lymphoproliferative disorder and/or hemophagocytic syndrome. Pathol Int 1998;48:934-943.
Quintanilla-Martinez L, Kimura H, Jaffe ES. EBV-positive lymphoproliferative disorders of childhood. In. WHO Classification of Tumours of the Haematopoietic and Lymphoid Tissues. Lyon: International Agency for Research on Cancer; 2008; p 278-280.
Rosenberg SA, Restifo NP, Yang JC, Morgan RA, Dudley ME. Adoptive cell transfer: a clinical path to effective cancer immunotherapy. Nat.Rev.Cancer 2008;8:299-308.
Rossig C, Brenner MK. Genetic modification of T lymphocytes for adoptive immunotherapy. Mol.Ther. 2004;10:5-18. 25
Sadelain M, Riviere I, Brentjens R. Targeting tumours with genetically enhanced T lymphocytes. Nat.Rev.Cancer 2003;3:35-45.
Shaffer DR, Savoldo B, Yi Z, et al. T cells redirected against CD70 for the immunotherapy of CD70-positive malignancies. Blood 2011;117:4304-4314.
Tey SK, Dotti G, Rooney CM, Heslop HE, Brenner MK. Inducible caspase 9 suicide gene to improve the safety of allodepleted T cells after haploidentical stem cell transplantation. Biol.Blood Marrow Transplant. 2007;13:913-924.
Till BG, Jensen MC, Wang J et al. Adoptive immunotherapy for indolent non-Hodgkin lymphoma and mantle cell lymphoma using genetically modified autologous CD20-specific T cells. Blood 2008;112:2261-2271.
van Oosterwijk MF, Juwana H, Arens R et al. CD27-CD70 interactions sensitise naive CD4+ T cells for IL-12-induced Th1 cell development. Int.Immunol. 2007;19:713-718.
Vera J, Savoldo B, Vigouroux S et al. T lymphocytes redirected against the kappa light chain of human immunoglobulin efficiently kill mature B lymphocyte-derived malignant cells. Blood 2006;108:3890-3897.
Yamamoto H, Kishimoto T, Minamoto S. NF-{kappa}B Activation in CD27 Signaling: Involvement of TNF Receptor-Associated Factors in Its Signaling and Identification of Functional Region of CD27. The Journal of Immunology 1998;161:4753-4759.
Yang ZZ, Novak AJ, Ziesmer SC, Witzig TE, Ansell SM. CD70+ non-Hodgkin lymphoma B cells induce Foxp3 expression and regulatory function in intratumoral CD4+CD25 T cells. Blood 2007;110:2537-2544.
Zhang T, Barber A, Sentman CL. Generation of antitumor responses by genetic modification of primary human T cells with a chimeric NKG2D receptor. Cancer Res 2006;66:5927-5933.

Although the present invention and its advantages have been described in detail, it should be understood that various changes, substitutions and alterations can be made herein without departing from the spirit and scope of the invention as defined by the appended claims. Moreover, the scope of the present application is not intended to be limited to the particular embodiments of the process, machine, manufacture, composition of matter, means, methods and steps described in the specification. As one of ordinary skill in the art will readily appreciate from the disclosure of the present invention, processes, machines, manufacture, compositions of matter, means, methods, or steps, presently existing or later to be developed that perform substantially the same function or achieve substantially the same result as the corresponding embodiments described herein may be utilized according to the present invention. Accordingly, the appended claims are intended to include within their scope such processes, machines, manufacture, compositions of matter, means, methods, or steps.

### FURTHER EMBODIMENTS

1. A chimeric antigen receptor that recognizes the CD70 antigen and that comprises an intracellular signaling domain.
2. The receptor of item 1, wherein the receptor is present on a T cell.
3. The receptor of item 1, further defined as comprising a CD70 receptor.
4. The receptor of item 1, wherein the CD70 receptor is CD27.
5. The receptor of item 1, wherein the intracellular signaling domain is the T-cell receptor CD3-ζ chain.
6. A method of targeting a cell having a CD70 antigen, comprising the steps of providing to the cell having the CD70 antigen a cell comprising the receptor of item 1.
7. The method of item 6, wherein the cell having the CD70 antigen is a cancer cell or a cell associated with an autoimmune disease.
8. The method of item 7, wherein the cancer cell is a hematological malignant cells.
9. The method of item 7, wherein the cancer cell is a lymphoma cell, leukemia cell, renal cell carcinoma cell, myeloma cell, or gliobastoma cell.
10. The method of item 7, wherein the cancer cell is a HTLV-1-associated malignant cell or a EBV-associated malignant cell.
11. The method of item 7, wherein the cancer cell is a solid tumor cell.
12. The method of item 11, wherein the solid tumor cell is a renal cancer cell, pancreatic cancer cell, ovarian cancer cell, lung cancer cell, nasopharyngeal cancer cell, thymic cancer cell, kidney cancer cell, pancreatic cancer cell, larynx cancer cell, pharynx cancer cell, skin cancer cell, ovarian cancer cell, lung cancer cell, colon cancer cell, breast cancer cell, or brain cancer cell.
13. The method of item 7, wherein cell the autoimmune disease is rheumatoid arthritis, arthritis, inflammation, autoimmune encephalitis, inflammatory bowel disease, colitis, or lupus.
14. The method of item 6, wherein the cell comprising the receptor of item 1 is a T cell.
15. The method of item 14, wherein the T cell is from the individual and is genetically modified to comprise the chimeric antigen receptor.
16. The method of item 14, wherein the T cell is not from the individual and is genetically modified to comprise the chimeric antigen receptor.
17. A method of treating a CD70-positive malignant cell in an individual, comprising the step of targeting the CD70-positive malignant cell with a tumor-specific T cell that comprises the chimeric antigen receptor of item 1.
18. The method of item 17, further comprising the step of modifying a T cell to harbor the chimeric antigen receptor of item 1.
19. The method of item 17, wherein the individual has received or is receiving or will receive an additional anti-cancer therapy.
20. The method of item 19, wherein the additional anti-cancer therapy comprises surgery, radiation, chemotherapy, immunotherapy, or hormone therapy.
21. A kit for treating an individual for cancer, comprising a polynucleotide that comprises an expression construct that encodes the chimeric receptor of item 1, said polynucleotide housed in a suitable container.
22. The kit of item 19, further comprising T cells.

## Claims

1. A nucleic acid sequence encoding a chimeric antigen receptor for use in a method of treating a solid tumor:
wherein the chimeric antigen receptor recognizes the CD70 antigen and comprises one or more intracellular signaling domains selected from CD3 zeta chain, OX40, CD28 and 4-1BB.

2. The nucleic acid sequence for use of claim 1, operably linked to a promoter.

3. The nucleic acid sequence for use of claim 2, wherein the promoter is constitutive or inducible.

4. The nucleic acid sequence for use of claim 1, 2, or 3, wherein the nucleic acid sequence is comprised in a vector.

5. The nucleic acid sequence for use of claim 4, wherein the vector is a viral vector.

6. The nucleic acid sequence for use of claim 5, wherein the viral vector is a retroviral vector, adenoviral vector, adeno-associated viral vector, or lentiviral vector.

7. The nucleic acid sequence for use of claim 4, wherein the vector is a plasmid.

8. The nucleic acid sequence for use of any one of claims 1-7, wherein the nucleic acid sequence is comprised in a cell.

9. A method of producing a nucleic acid sequence encoding a chimeric antigen receptor that recognizes the CD70 antigen and that comprises one or more intracellular signaling domains selected from the group consisting of OX40, CD28 and 4-1BB, comprising the steps of:
using recombinant DNA technique to prepare a construct that encodes the chimeric receptor; and
introducing the construct into a vector.

10. The method of claim 9, wherein the construct comprises a promoter that is operably linked to the sequence encoding the chimeric antigen receptor.

11. The method of claim 10, wherein the promoter is constitutive or inducible.

12. The method of any one of claims 9-11, wherein the vector is a viral vector.

13. The method of claim 12, wherein the viral vector is a retroviral vector, adenoviral vector, adeno-associated viral vector, or lentiviral vector.

14. The method of claim 9, wherein the vector is a plasmid.

15. The method of any one of claims 9-14, wherein the nucleic acid sequence is comprised in a cell, optionally wherein the cell is a T cell.
